# EUROPÄISCHE PATENTSCHRIFT

(11) **EP 2 593 447 B1**
(45) Veröffentlichungstag und Bekanntmachung des Hinweises auf die Patenterteilung: **17.08.2016**
(21) Anmeldenummer: 11736039.6
(22) Anmeldetag: 13.07.2011
(51) Int. Cl.: C07D 401/04, C07D 405/04, C07D 409/04, A01N 43/40

(54) **3-PYRIDYL-HETEROARYLCARBOXAMIDVERBINDUNGEN ALS SCHÄDLINGSBEKÄMPFUNGSMITTEL**
3-PYRIDYL-HETEROARYLCARBOXAMIDE COMPOUNDS AS PESTICIDES
COMPOSÉS DE 3-PYRIDYL-HETEROARYLCARBOXAMIDE COMME D'AGENTS DE LUTTE CONTRE LES NUISIBLES

(30) Priorität: 15.07.2010 US 364514 P; 15.07.2010 EP 10169682
(43) Veröffentlichungstag der Anmeldung: 22.05.2013
(73) Patentinhaber: Bayer Intellectual Property GmbH, 40789 Monheim (DE)
(72) Erfinder: MÜHLTHAU, Friedrich, August, 65812 Bad Soden am Taunus (DE); BRETSCHNEIDER, Thomas, 53797 Lohmar (DE); FISCHER, Reiner, 40789 Monheim (DE); FÜßLEIN, Martin, 40225 Düsseldorf (DE); HEIL, Markus, 42799 Leichlingen (DE); HENSE, Achim, 51379 Leverkusen (DE); KLUTH, Joachim, 40764 Langenfeld (DE); KÖHLER, Adeline, 40764 langenfeld (DE); FRANKEN, Eva-Maria, 51381 Leverkusen Bergisch Neukirchen (DE); MALSAM, Olga, 51503 Rösrath (DE); VOERSTE, Arnd, 50674 Köln (DE); JESCHKE, Peter, 51467 Bergisch Gladbach (DE); BECKER, Angela, 40235 Düsseldorf (DE); LÖSEL, Peter, 51371 Leverkusen (DE); SATO, Yoshitaka, Ibaraki 3002648 (JP)
(74) Vertreter: BIP Patents
(86) Internationale Anmeldenummer: PCT/EP2011/061949
(87) Internationale Veröffentlichungsnummer: WO 2012/007500

(56) Entgegenhaltungen:
- EP-A1- 1 710 233
- WO-A1-03/092686
- WO-A1-2004/019947
- WO-A1-2005/030206
- WO-A1-2005/061494
- WO-A1-2005/061495
- WO-A1-2005/061510
- WO-A1-2006/118267
- WO-A1-2009/149858
- US-A- 4 863 947
- US-A1- 2002 013 326
- US-A1- 2003 069 290
- US-A1- 2003 162 812
- US-A1- 2008 171 741
- US-A1- 2009 143 228
- US-A1- 2009 170 913
- US-B1- 6 323 227

## Beschreibung

Die vorliegende Anmeldung betrifft neue heterocyclische Verbindungen, Verfahren zu Ihrer Herstellung und ihre Verwendung zur Bekämpfung von tierischen Schädlingen, zu denen Arthropoden und insbesondere Insekten zählen.

Bestimmte heterocyclische Verbindungen sind bereits als insektizid wirksame Verbindungen bekannt geworden (vgl. WO1999/006380 A1, EP 0 097 126 B1, US 2003/162812 A1, US 2002/013326 A1, WO 2009/149858 A1).

Bestimmte heterocyclische Verbindungen sind bereits als herbizid wirksame Verbindungen bekannt geworden (vgl. WO 2005/047281 A1).

Ferner sind folgende Verbindungen bekannt geworden, für die aber keine insektizide Wirkung beschrieben wurde:

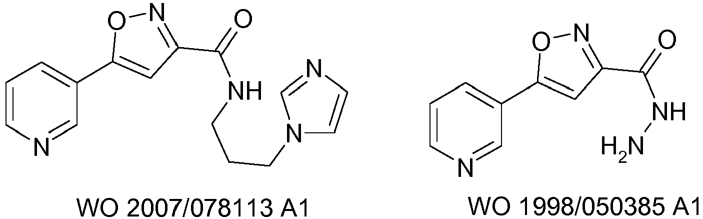

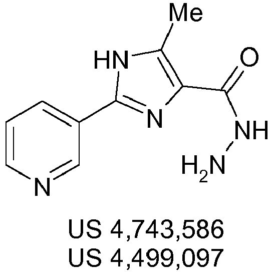

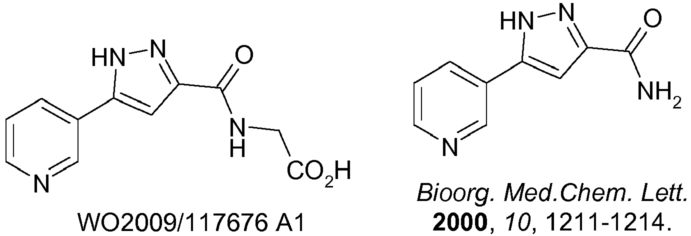

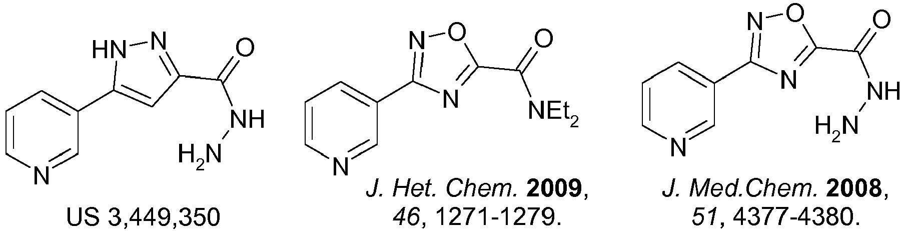

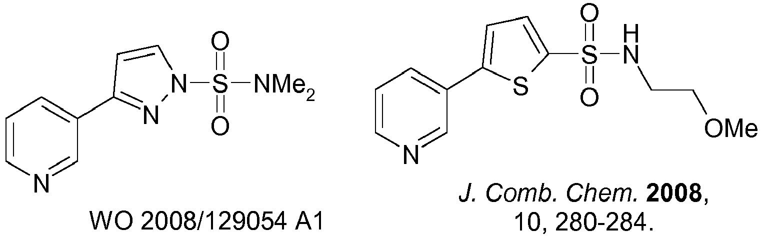

In WO 2000/027823 A1, US 6,288,061 B1, WO 1999/033827 A1, DE 28 38 892 A1, Chem. Heterocycl. Comp. 2000, 36, 1226-1231, WO 2000/021959 A1, EP 0647 635 A1, Pharmazie 1989, 44, 191-193, EP 0 097 126 B1, DE 24 53 082 A1, DE 24 53 083 A1 und US 4,110,456 werden heterocyclische Verbindungen offenbart, für die pharmazeutische Verwendungen angegeben werden.

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch die Verwendung von Verbindungen der Formel (I) 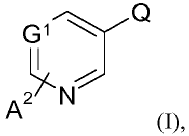
- G¹: für C-A¹ steht,
- A¹: für Wasserstoff steht,
- A²: für Wasserstoff steht,
- Q: für einen der folgenden Reste 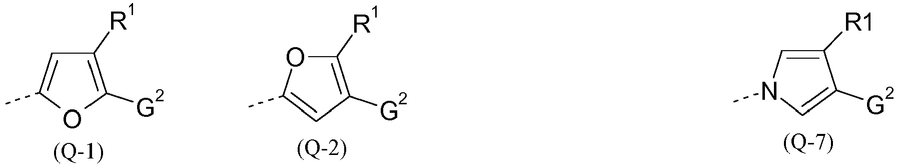
steht,
- R¹: für Wasserstoff, C₁-C₆-Alkyl, C₃-C₆-Cycloalkyl, Halogen, Cyano, C₁-C₃-Haloalkyl, Hydroxy oder C₁-C₆-Alkoxy steht,
- G²: für C(=X)NR³R⁴ steht,
- X: für Sauerstoff oder Schwefel steht,
- R³: für einen Rest aus der Reihe Wasserstoff, C₁-C₆-Alkyl, C₃-C₆-Cycloalkyl, C₁-C₆-Halogenalkyl, Cyano-C₁-C₆-alkyl, C₁-C₆-Alkoxy, C₁-C₆-Halogenalkoxy, C₂-C₆-Alkenyl, C₁-C₆-Alkoxy-C₁-C₆-alkyl, gegebenenfalls durch Halogen substituiertes C₁-C₆-Alkylcarbonyl, gegebenenfalls durch Halogen substituiertes C₁-C₆-Alkoxycarbonyl, gegebenenfalls durch Halogen, C₁-C₆-Alkyl, C₁-C₆-Alkoxy, C₁-C₆-Halogenalkyl und Cyano substituiertes C₃-C₆-Cycloalkylcarbonyl, oder für ein Kation, wie beispielsweise ein ein- oder zweiwertiges Metallion oder ein gegebenenfalls durch C₁-C₆-Alkyl oder Aryl-C₁-C₆-alkyl substituiertes Ammonium-Ion steht,
- R⁴: für einen Rest aus der Reihe Wasserstoff, Cyano, jeweils gegebenenfalls durch Halogen, Cyano, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl und C₁-C₆-Halogenalkylsulfonyl substituiertes C₁-C₆-Alkyl, C₂-C₆-Alkenyl und C₂-C₆-Alkinyl, C₁-C₆-Alkoxy, C₁-C₆-Halogenalkoxy, gegebenenfalls durch Halogen substituiertes Bis(C₁-C₆-alkoxy)-C₁-C₆-alkyl, gegebenenfalls durch Halogen substituiertes Bis(C₁-C₆-alkylthio)-C₁-C₆-alkyl, gegebenenfalls durch Halogen substituiertes C₁-C₆-Alkylcarbonyl-C₁-C₆-alkyl, gegebenenfalls durch Halogen substituiertes C₁-C₆-Alkylsulfinyl-C₁-C₆-alkyl, gegebenenfalls durch Halogen substituiertes C₁-C₆-Alkylsulfonyl-C₁-C₆-alkyl, gegebenenfalls durch Halogen substituiertes C₁-C₆-Alkoxycarbonyl-C₁-C₆-alkyl, C₂-C₄-Alkinyloxy, gegebenenfalls durch Halogen substituiertes C₃-C₆-Cycloalkylcarbonyl, gegebenenfalls durch Halogen substituiertes C₃-C₆-Cycloalkyl-C₁-C₆-alkyl, jeweils gegebenenfalls durch Halogen, C₁-C₆-Alkyl, C₁-C₆-Halogenalkyl, C₁-C₆-Alkoxy oder C₁-C₆-Halogenalkoxy substituiertes C₃-C₆-Cycloalkyl und C₃-C₆-Cycloalkenyl, in welchen die Ringe ein bis drei Heteroatome aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Aryl, Heteroaryl, Heterocyclyl, Heterocyclyl-C₁-C₆-alkyl, Aryl-C₁-C₆-alkyl, Heteroaryl-C₁-C₆-alkyl, oder für NR⁵R⁶ steht, worin R⁵ und R⁶ unabhängig voneinander für einen Rest aus der Reihe Wasserstoff, C₁-C₆-Alkyl, C₁-C₆-Haloalkyl, C₃-C₆-Cycloalkyl, C₁-C₆-Alkoxy, C₁-C₆-Alkylcarbonyl, C₁-C₆-Alkoxylcarbonyl, Hetaryl und Heterocyclyl stehen oder R⁵ und R⁶ gemeinsam mit dem Stickstoffatom, an das sie gebunden sind, einen gegebenenfalls durch Halogen substituierten Heterocyclus bilden,
- R³ und R⁴: auch gemeinsam mit dem Stickstoffatom, an das sie gebunden sind, einen gegebenenfalls durch C₁-C₆-Alkyl, C₁-C₆-Alkoxy oder Halogen substituierten und gegebenenfalls ein oder zwei Heteroatome aus der Reihe Sauerstoff, Stickstoff und Schwefel enthaltenden 3- bis 7-gliedrigen Ring bilden können, wobei Sauerstoffatome nicht unmittelbar benachbart sein dürfen,
sowie Salzen, N-Oxiden und tautomeren Formen der Verbindungen der Formel (I), wobei
Halogen ausgewählt ist aus der Reihe Fluor, Chlor, Brom und Iod,
Aryl (auch als Teil einer größeren Einheit, wie beispielsweise Arylalkyl) ausgewählt ist aus der Reihe Phenyl, Naphthyl, Anthryl, Phenanthrenyl und
Hetaryl (gleichbedeutend mit Heteroaryl, auch als Teil einer größeren Einheit, wie beispielsweise Hetarylalkyl) ausgewählt ist aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
zur Bekämpfung von tierischen Schädlingen.

Die Erfindung betriffe ferner neue Verbindungen der Formel (I) 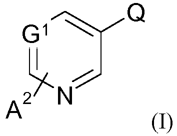 worin
- G¹: für C-A¹ steht,
- A¹: für Wasserstoff steht,
- A²: für Wasserstoff steht,
- Q: für einen der folgenden Reste

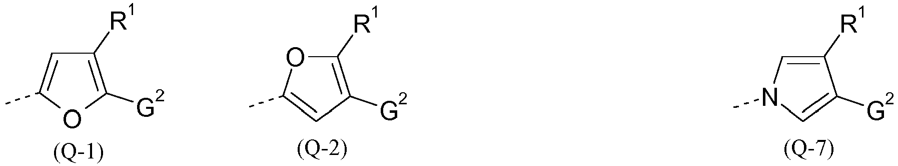

- R¹: für Wasserstoff, C₁-C₄-Alkyl, C₃-C₆-Cycloalkyl, Halogen, Cyano, C₁-C₃-Haloalkyl, Hydroxy oder C₁-C₄-Alkoxy steht,
- G²: für C(=X)NR³R⁴ steht,
- X und Y: unabhängig voneinander für Sauerstoff oder Schwefel stehen,
- R³: für einen Rest aus der Reihe Wasserstoff, C₁-C₄-Alkyl, C₃-C₆-Cycloalkyl, C₁-C₄-Halogenalkyl, Cyano-C₁-C₄-alkyl, C₁-C₄-Alkoxy, C₁-C₄-Halogenalkoxy, C₂-C₄-Alkenyl, C₁-C₄-Alkoxy-C₁-C₄-alkyl, gegebenenfalls durch Halogen substituiertes C₁-C₄-Alkylcarbonyl, gegebenenfalls durch Halogen substituiertes C₁-C₄-Alkoxycarbonyl, gegebenenfalls durch Halogen, C₁-C₄-Alkyl, C₁-C₄-Alkoxy, C₁-C₄-Halogenalkyl und Cyano substituiertes C₃-C₆-Cycloalkylcarbonyl, oder für ein Kation, wie beispielsweise ein ein- oder zweiwertiges Metallion oder ein gegebenenfalls durch C₁-C₄-Alkyl oder Aryl-C₁-C₄-alkyl substituiertes Ammonium-Ion steht,

- R⁴: für einen Rest aus der Reihe Wasserstoff, Cyano, jeweils gegebenenfalls durch Halogen, Cyano, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl und C₁-C₄-Halogenalkylsulfonyl substituiertes C₁-C₄-Alkyl, C₂-C₄-Alkenyl und C₂-C₄-Alkinyl, C₁-C₄-Alkoxy, C₁-C₄-Halogenalkoxy, gegebenenfalls durch Halogen substituiertes Bis(C₁-C₄-alkoxy)-C₁-C₄-alkyl, gegebenenfalls durch Halogen substituiertes Bis(C₁-C₄-alkylthio)-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, gegebenenfalls durch Halogen substituiertes C₁-C₄-Alkoxycarbonyl-C₁-C₄-alkyl, C₂-C₄-Alkinyloxy, gegebenenfalls durch Halogen substituiertes C₃-C₆-Cycloalkyl-carbonyl, gegebenenfalls durch Halogen substituiertes C₃-C₆-Cycloalkyl-C₁-C₄-alkyl, jeweils gegebenenfalls durch Halogen, C₁-C₄-Alkyl, C₁-C₄-Halogenalkyl, C₁-C₄-Alkoxy oder C₁-C₄-Halogenalkoxy substituiertes C₃-C₆-Cycloalkyl und C₃-C₆-Cycloalkenyl, in welchen die Ringe ein bis drei Heteroatome aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₄-Alkyl, C₁-C₄-Halogenalkyl,C₃-C₆-Cycloalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Aryl, Heteroaryl, Aryl-C₁-C₄-alkyl, Heteroaryl-C₁-C₄-alkyl, oder für NR⁵R⁶ steht, worin R⁵ und R⁶ unabhängig voneinander für einen Rest aus der Reihe Wasserstoff, C₁-C₄-Alkyl, C₁-C₄-Haloalkyl, C₃-C₆-Cycloalkyl, C₁-C₄-Alkoxy, C₁-C₄-Alkylcarbonyl, C₁-C₄-Alkoxylcarbonyl, Hetaryl und Heterocyclyl stehen oder R⁵ und R⁶ gemeinsam mit dem Stickstoffatom, an das sie gebunden sind, einen gegebenenfalls durch Halogen substituierten Heterocyclus bilden,
- R³ und R⁴: auch gemeinsam mit dem Stickstoffatom, an das sie gebunden sind, einen gegebenenfalls durch C₁-C₄-Alkyl, C₁-C₄-Alkoxy oder Halogen substituierten und gegebenenfalls ein oder zwei Heteroatome aus der Reihe Sauerstoff, Stickstoff und Schwefel enthaltenden 3- bis 7-gliedrigen Ring bilden können, wobei Sauerstoffatome nicht unmittelbar benachbart sein dürfen,
und wobei
Halogen ausgewählt ist aus der Reihe Fluor, Chlor, Brom und Iod,
Aryl (auch als Teil einer größeren Einheit, wie beispielsweise Arylalkyl) ausgewählt ist aus der Reihe Phenyl, Naphthyl, Anthryl, Phenanthrenyl und
Hetaryl (auch als Teil einer größeren Einheit, wie beispielsweise Hetarylalkyl) ausgewählt ist aus der Reihe Thienyl, Pyrimidyl, Oxadiazolyl, Oxazolyl, Pyrazinyl, Imidazolyl, Thiazolyl und Furanyl.
Bevorzugt sind Verbindungen der Formel (I), worin
- G¹: für C-A¹ steht,
- A¹: für Wasserstoff steht,
- A²: für Wasserstoff steht,
- Q: für Q-1, Q-2 oder Q-7 steht,
- R¹: für Wasserstoff oder C₁-C₄-Alkyl steht,
- G²: für C(=X)NR³R⁴ steht,
- X: für Sauerstoff steht,
- R³: für einen Rest aus der Reihe Wasserstoff und C₁-C₄-Alkyl steht,
- R⁴: für einen Rest aus der Reihe Wasserstoff, jeweils gegebenenfalls durch Halogen, Cyano und C₁-C₄-Alkylthio substituiertes C₁-C₄-Alkyl und C₂-C₄-Alkenyl, für C₂-C₄-Alkinyl, gegebenenfalls durch Halogen substituiertes C₃-C₆-Cycloalkyl-C₁-C₄-alkyl und gegebenenfalls durch C₁-C₄-Halogenalkyl substituiertes Heteroaryl-C₁-C₄-alkyl steht und
- R³ und R⁴: auch gemeinsam mit dem Stickstoffatom, an das sie gebunden sind, einen 3- bis 7-gliedrigen Ring bilden können, der keine weiteren Heteroatome enthält und wobei
Halogen ausgewählt ist aus der Reihe Fluor, Chlor, Brom und Iod und
Hetaryl (auch als Teil einer größeren Einheit, wie beispielsweise Hetarylalkyl) ausgewählt ist aus der Reihe Thienyl, Pyrimidyl und Thiazolyl.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

In einer bevorzugten Ausführungsform betrifft die Erfindung Verbindungen der Formel (I-1a) 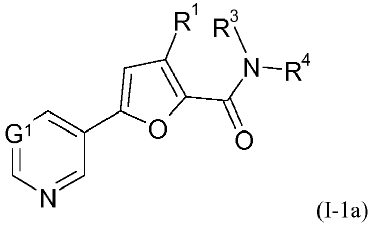 worin G¹, R¹, R³ und R⁴ die oben angegebene Bedeutung haben.

In einer weiteren bevorzugten Ausführungsform betrifft die Erfindung Verbindungen der Formel (I-2a) 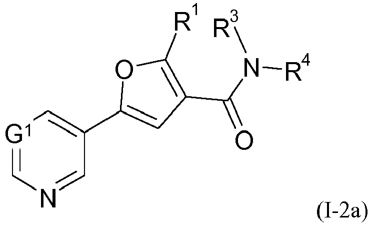 worin G¹, R¹, R³ und R⁴ die oben angegebene Bedeutung haben.

In einer weiteren bevorzugten Ausführungsform betrifft die Erfindung Verbindungen der Formel (I-7a) 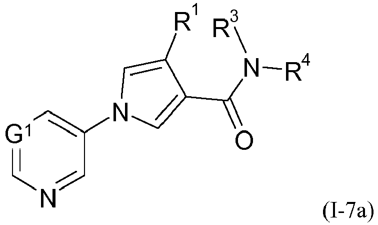 worin G¹, R¹, R³ und R⁴ die oben angegebene Bedeutung haben.

Die erfindungsgemäßen Verbindungen können nach üblichen, dem Fachmann bekannten Methoden hergestellt werden.

Die Herstellung der heterocyclischen Grundgerüste in Verbindungen der Formel (I) ist nach einer oder mehreren Synthesevarianten, die in den Reaktionsschemata 1 bis 4 und 6 gezeigt werden, möglich.

Verbindungen der Formel (I), in denen Q für Q-1, Q-2 oder Q-7 steht, sind beispielsweise nach Reaktionsschema 1 darstellbar.

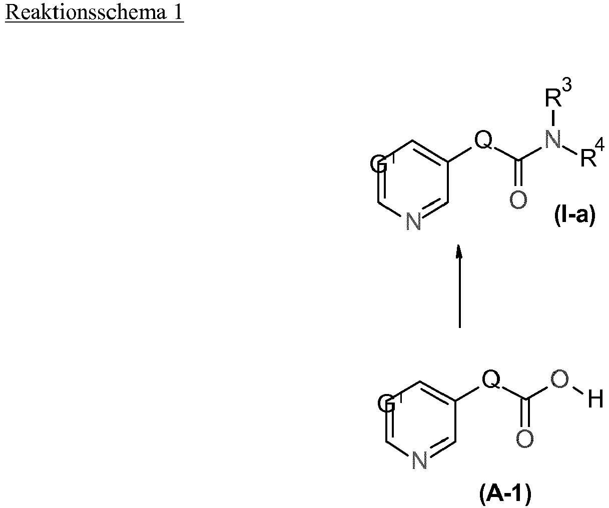

Verbindungen der Formel (I-a) lassen sich herstellen, indem man die Carbonsäuren der Formel (A-1) durch Überführung in Säurechloride, Säurefluoride oder gemischte Anhydride oder durch Umsetzung mit Kupplungsreagenzien wie zum Beispiel BOPCI (vgl. z. B. Houben-Weyl, Methoden der organischen Chemie, Band 15/2; M. Bodanszky et al., The Practice of Peptide Synthesis, Springer, New York, 1984; C. A. G. N. Montalbetti et al., Tetrahedron 2005, 61, 10827-10852) aktiviert und anschließend mit den entsprechenden Aminen gegebenenfalls in Gegenwart eines Kupplungsreagens und eines basischen Reaktionshilfsmittels umsetzt. Kupplungsreagenzien, die sich zu Herstellung von Amidbindungen eignen, sind bekannt (vgl. Bodansky et al., Peptide Synthesis 2nd ed, Wiley & Sons, New York, 1976).

2-Brom-2,2-difluorethanamin kann nach allgemein bekannten Methoden erhalten werden (vgl. WO 2004/916113 A2).

Verbindungen der Formel (A-1), in denen Q für Q-1, Q-2 oder Q-7 steht, sind beispielsweise nach Reaktionsschema 2 darstellbar.

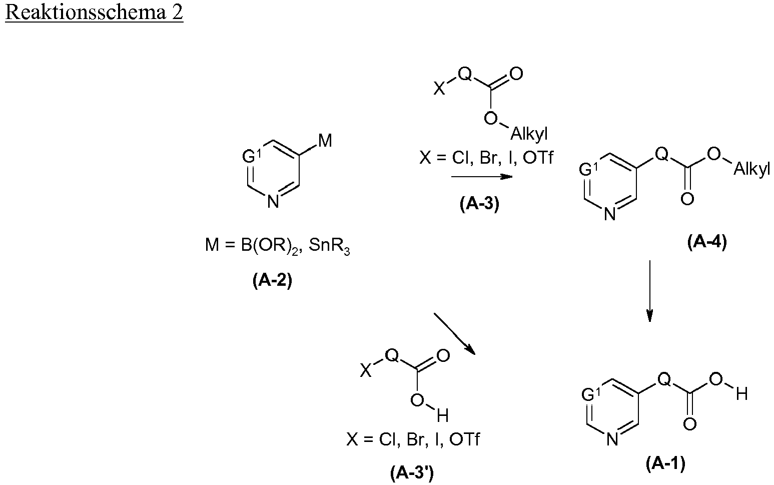

Zur Herstellung der erfindungsgemäßen Verbindungen werden zunächst (Hetero)arylboronsäuren oder (Hetero)arylboronsäureester der Formel (A-2) mit den entsprechenden halogenierten Estern der Formel (A-3) nach bekannten Methoden (Chem. Rev. 1995, 95, 2457-2483; Tetrahedron 2002, 58, 9633-9695; Metal-Catalyzed Cross-Coupling Reactions (Eds.: A. de Meijere, F. Diederich), 2nd ed., Wiley-VCH, Weinheim, 2004**)** unter Übergangsmetallsalzkatalyse zu Verbindungen der Formel (A-4) umgesetzt. Zum Beispiel ist die Umsetzung von Pyridin-3-ylboronsäure mit Methyl-5-brom-2-furanat [Q-1] beschrieben (vgl. L. A. McAllister et al., J. Am. Chem. Soc. 2006, 128, 4176-4177). Analoge Umsetzungen mit substituierten Thiophenen sind in WO 2005/61494 und Z.-K. Wan et al., Bioorg. Med. Chem. Lett. 2006, 16, 4941-4945 beschrieben. Ähnliche Kupplungs-Reaktionen sind für folgende teilweise substituierten halogenierten Carbonsäureester der Formel (A-3) oder der analogen Säuren der Formel (A-3') beschrieben: Methyl-5-chlor-3-furanat [US 6,302,047 B1, Q-2].

Entsprechende Reaktionen sind auch mit Trialkylzinnheteroarylverbindungen beschrieben ( vgl. J. Zhang et al., Bioorg. Med. Chem. Lett. 2000, 10, 2575-2578).

Boronsäuren und Boronsäureester der Formel (A-2) sind allgemein bekannt und mit allgemein bekannten Methoden herstellbar [Pyridin-3-ylboronsäure (vgl. WO 2005/66162), Pyrimidin-5-ylboronsäure (vgl. WO 2005/103019), 3-(4,4,5,5-Tetramethyl-1,3,2-dioxaborolan-2-yl)pyridin (vgl. T. Ishiyama et al., Tetrahedron 2001, 57, 9813-9816)]. Trialkylzinnheteroarylverbindungen der Formel (A-2) sind allgemein bekannt und mit allgemein bekannten Methoden herstellbar [3-(Trimethylstannyl)pyridin (vgl. US 6,544,985, WO 2003/72553). Halogenierte Carbonsäureester der Formel (A-3) oder deren Säuren sind allgemein bekannt und mit allgemein bekannten Methoden herstellbar [Ethyl-5-brom-2-furanat (vgl. WO 2004/33440), Methyl-5-brom-3-furanat (vgl. G. Johansson et al., J. Med. Chem. 1997, 40, 3804-3819), Methyl-5-bromthiophen-2-carboxylat (vgl. WO 2005/79791), Methyl-2-chlor-1,3-oxazol-5-carboxylat (vgl. WO 2007/131953), Ethyl-3-brom-1,2-oxazol-5-carboxylat (vgl. WO 2005/26149)].

Verbindungen der Formel (A-4), worin Alkyl bevorzugt für Methyl oder Ethyl steht, lassen sich durch Esterspaltung des Carbonsäureesters in die Verbindung der Formel (A-1) überführen. Die Esterspaltung kann mit den bekannten Verfahren durchgeführt werden (vgl. Greene's protective groups in organic synthesis, 4th ed., P.G.M. Wuts, T.W. Greene, John Wiley & Sons, Inc., Hoboken, New Jersey, 2007); beispielsweise kann die Verbindung der Formel (A-4) mit wässriger Lithiumhydroxidlösung in Tetrahydrofuran und anschließendem Ansäuern oder mit wässriger Natriumhydroxidlösung in Alkoholen und anschließendem Ansäuern zu der entsprechenden Säure der Formel (A-1) umgesetzt werden.

Verbindungen der Formel (A-1), in denen Q für Q-1 oder Q-2 steht, sind beispielsweise nach Reaktionsschema 3 darstellbar.

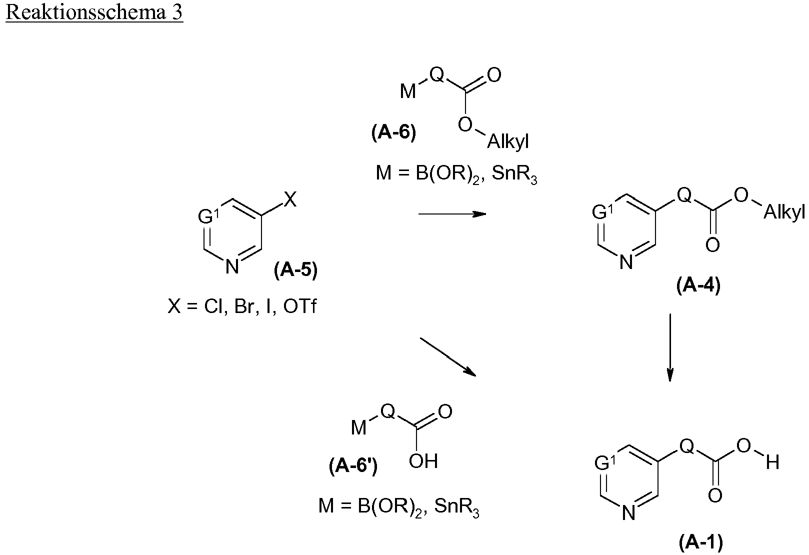

Zur Herstellung der erfindungsgemäßen Verbindungen werden zunächst Halogenide oder Triflate (A-5) mit den entsprechenden (Hetero)arylboronsäuren oder (Hetero)arylboronsäureestern der Formel (A-6) oder (A-6') nach bekannten Methoden (siehe Reaktionsschema 2) unter Übergangsmetallsalzkatalyse zu Verbindungen der Formel (A-4) oder (A-1) umgesetzt.

Entsprechende Reaktionen sind auch mit Trialkylzinnheteroarylverbindungen beschrieben Bioorg. Med. Chem. 2003, 11, 281-292 [Q-1]).

Die Umsetzung von Estern der Formel (A-4) zu Carbonsäuren der Formel (A-1) kann wie in Reaktionsschema 3 beschrieben durchgeführt werde.

Verbindungen der Formel (A-1), in denen Q für Q-7 steht, sind beispielsweise nach Reaktionsschema 4 darstellbar.

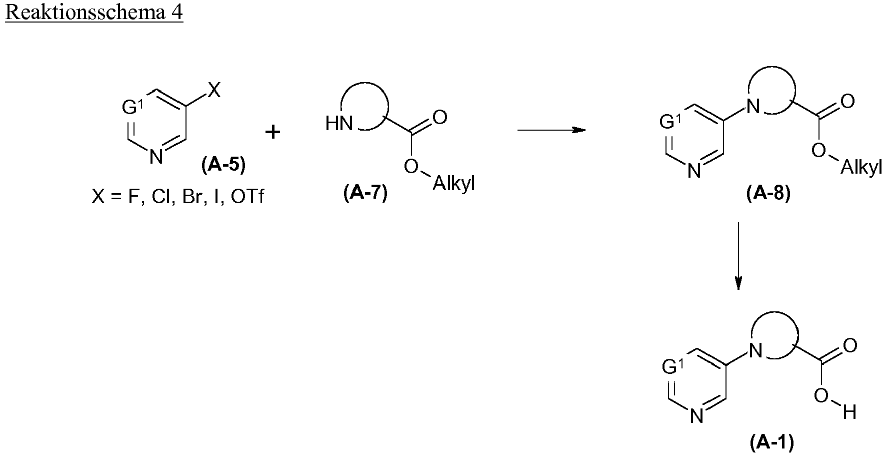

Zur Herstellung der erfindungsgemäßen Verbindungen werden zunächst Halogenide oder Triflate der Formel (A-5) mit den Estern der Formel (A-7) nach bekannten Methoden (Mini-Reviews in Organic Chemistry 2008, 5, 323-330; Molecules 2009, 14, 5169-5178; Pyrazol: Eur. J. Org. Chem. 2004, 4, 695-709) unter Übergangsmetallsalzkatalyse zu Verbindungen der Formel (A-8) umgesetzt. Zum Beispiel ist die Umsetzung von Methyl-1H-pyrrol-3-carboxylat [Q-7] mit 4-Bromisochinolin beschrieben (vgl. WO 2004/7478).

Die Umsetzung von Estern der Formel (A-8) zu Carbonsäuren der Formel (A-1) kann wie in Reaktionsschema 3 beschrieben durchgeführt werden.

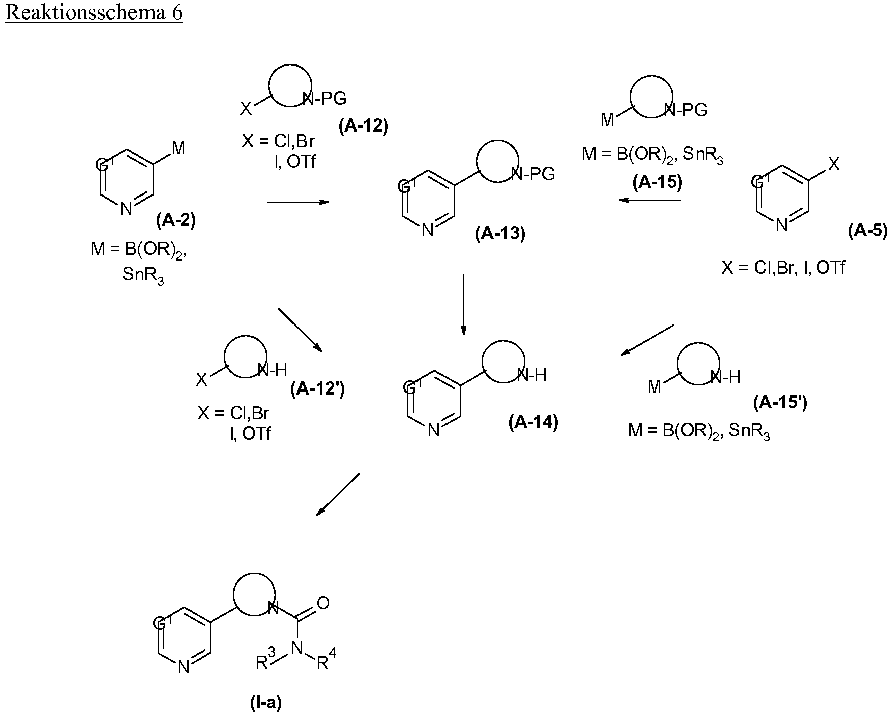

Zur Herstellung der erfindungsgemäßen Verbindungen vom Typ (I-a) werden zunächst, analog zu den in Reaktionsschema 2 gezeigten Umsetzungen, (Hetero)arylboronsäuren oder (Hetero)arylboronsäureester der Formel (A-2) mit den entsprechenden halogenierten Verbindungen (A-12, PG entspricht beispielsweise Trityl) oder der Formel (A-12') nach bekannten Methoden *(vide supra)* unter Übergangsmetallsalzkatalyse zu Verbindungen der Formeln (A-13) bzw. (A-14) umgesetzt. Die Umsetzung folgender Verbindung mit substituierten Pyridinylboronsäuren ist beschrieben: 3-Brom-1-(triisopropylsilyl)-1H-pyrrol (J. Am. Chem. Soc. 2007, 129, 3358-3366, Q-7).

Alternativ können Verbindungen der Formeln (A-13) bzw. (A-14), analog zu den in Reaktionsschema 3 gezeigten Umsetzungen, durch Übergangsmetallsalz-katalysierte Umsetzung von Halogeniden oder Triflaten der Formel (A-5) mit den entsprechenden (Hetero)arylboronsäuren oder (Hetero)aryl-boronsäureester der Formeln (A-15) oder (A-15') hergestellt werden.

Die Umsetzung der Verbindung der Formel (A-13) zu der ungeschützten Verbindung der Formel (A-14) kann nach allgemein bekannten Methoden durchgeführt werden (vgl. Greene's protective groups in organic synthesis, 4th ed., P.G.M. Wuts, T.W. Greene, John Wiley & Sons, Inc., Hoboken, New Jersey, 2007); beispielsweise kann (A-X, PG = Trityl) mit Salzsäure in Ethanol zu der entsprechenden ungeschützten Verbindung der Formel (A-14) umgesetzt werden (vgl. M. T. Burger et al., J. Med. Chem. 2006, 49, 1730-1743).

Zur Herstellung der erfindungsgemäßen Verbindungen vom Typ (I-a,) werden die entsprechenden Verbindungen der Formel (A-14) mit den entsprechenden Acylierungsreagentien gegebenenfalls in Gegenwart eines Aktivators und eines basischen Reaktionshilfsmittels umgesetzt.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP-POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90%.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiziden, Düngemitteln, Lockstoffen, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des weiteren können solche Wirkstoffkombinationen das Pflanzenwachstum verbessern, die Toleranz gegenüber hohen oder niedrigen Temperaturen, gegen Trockenheit oder gegen Wasser- bzw. Bodensalzgehalt erhöhen, die Blühleistung steigern, die Ernte erleichtern und Ernteerträge steigern, die Reife beschleunigen, die Qualität und/oder den Ernährungswert der Ernteprodukte steigern, die Lagerfähigkeit verlängern und/oder die Bearbeitbarkeit der Ernteprodukte verbessern. Durch Kombination der erfindungsgemäßen Wirkstoffe mit Mischpartnern erhält man synergistische Effekte, d.h. die Wirksamkeit der jeweiligen Mischung ist größer als aufgrund der Wirksamkeiten der Einzelkomponenten zu erwarten war. Generell können die Kombinationen sowohl als Saatgutanwendungen als auch in Vor-, Tank- oder Fertigmischungen verwendet werden.

Jeder zusätzliche Wirkstoff kann in einem weiten Bereich, bevorzugt in einem Verhältnis von 100:1 bis 1.100, besonders bevorzugt von 5:1 bis 1:5 mit den erfindungsgemäßen Wirkstoffen gemischt werden.

Besonders günstige Mischungspartner sind z.B. die folgenden

### Insektizide / Akarizide / Nematizide:

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

### (1) Acetylcholinesterase (AChE) Inhibitoren, wie beispielsweise

Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
Organophosphate, z.B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.

### (2) GABA-gesteuerte Chlorid-Kanal-Antagonisten, wie beispielsweise

Organochlorine, z.B. Chlordane und Endosulfan (alpha-); oder
Fiprole (Phenylpyrazole), z.B. Ethiprole, Fipronil, Pyrafluprole und Pyriprole.

### (3) Natrium-Kanal-Modulatoren / Spannungsabhängige Natrium-Kanal-Blocker, wie beispielsweise

Pyrethroide, z.B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda), Cypermethrin (alpha-, beta-, theta-, zeta-), Cyphenothrin [(1R)-trans-Isomere], Deltamethrin, Dimefluthrin, Empenthrin [(EZ)-(1R)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1R)-trans-Isomer], Prallethrin, Profluthrin, Pyrethrine (pyrethrum), Resmethrin, RU 15525, Silafluofen, Tefluthrin, Tetramethrin [(1R)- Isomere], Tralomethrin, Transfluthrin und ZXI 8901; oder
DDT; oder Methoxychlor.

### (4) Nikotinerge Acetylcholin-Rezeptor-Agonisten, wie beispielsweise

Neonikotinoide, z.B. Acetamiprid, Clothianidin, Dinotefuran, Imidacloprid, Nitenpyram, Thiacloprid, Thiamethoxam; oder
Nikotin.

### (5) Allosterische Acetylcholin-Rezeptor-Modulatoren (Agonisten), wie beispielsweise

Spinosyne, z.B. Spinetoram und Spinosad.

### (6) Chlorid-Kanal-Aktivatoren, wie beispielsweise

Avermectine/Milbemycine, z.B. Abamectin, Emamectin-benzoate, Lepimectin und Milbemectin.
(7) Juvenilhormon-Analoge, z.B. Hydroprene, Kinoprene, Methoprene; oder Fenoxycarb; Pyriproxyfen.
(8) Wirkstoffe mit unbekannten oder nicht spezifischen Wirkmechanismen, wie beispielsweise Begasungsmittel, z.B. Methylbromid und andere Alkylhalogenide; oder
   Chloropicrin; Sulfurylfluorid; Borax; Brechweinstein.
(9) Selektive Fraßhemmer, z.B. Pymetrozine; oder Flonicamid.
(10) Milbenwachstumsinhibitoren, z.B. Clofentezine, Diflovidazin, Hexythiazox, Etoxazole.
(11) Mikrobielle Disruptoren der Insektendarmmembran, wie beispielsweise *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis,* und BT-Pflanzen-Proteine, z.B. Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin, Fenbutatin oxide; oder
   Propargite; Tetradifon.
(13) Entkoppler der oxidativen Phoshorylierung durch Unterbrechung des H-Protongradienten, wie beispielsweise Chlorfenapyr und DNOC.
(14) Nikotinerge Acetylcholin-Rezeptor-Antagonisten, wie beispielsweise Bensultap, Cartap (-Hydrochlorid), Thiocylam, und Thiosultap (-sodium).
(15) Inhibitoren der Chitinbiosynthese, Typ 0, wie beispielsweise Benzoylharnstoffe, z.B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, wie beispielsweise Cyromazine.
(18) Ecdysonagonisten/-disruptoren, wie beispielsweise
   Diacylhydrazine, z.B. Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; Acequinocyl; Fluacrypyrim.
(21) Komplex-I-Elektronentransportinhibitoren, beispielsweise aus der Gruppe der METI-Akarizide, z.B. Fenazaquin, Fenpyroximate, Pyrimidifen, Pyridaben, Tebufenpyrad, Tolfenpyrad; oder
   Rotenone (Derris).
(22) Spannungsabhängige Natriumkanal-Blocker, z.B. Indoxacarb; Metaflumizone.
(23) Inhibitoren der Acetyl-CoA-Carboxylase, wie beispielsweise Tetronsäure-Derivate, z.B. Spirodiclofen und Spiromesifen; oder Tetramsäure-Derivate, z.B. Spirotetramat.
(24) Komplex-IV-Elektronentransportinhibitoren, wie beispielsweise Phosphine, z.B. Aluminiumphosphid, Kalziumphosphid, Phosphin, Zinkphosphid; oder Cyanid.
(25) Komplex-II-Elektronentransportinhibitoren, wie beispielsweise Cyenopyrafen.
(28) Ryanodinrezeptor-Effektoren, wie beispielsweise Diamide, z.B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxylat (bekannt aus WO2007/043677).

Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chloro-2-[(3,4,4-trifluorobut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende Verbindungen:
4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Sulfoxaflor (ebenfalls bekannt aus WO2007/149134) und seine Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911),
1-[2-fluoro-4-methyl-5-[(2,2,2-trifluoroethyl)sulfinyl]phenyl]-3-(trifluoromethyl)-1H-1,2,4-Triazol-5-amine (bekannt aus WO 2006/043635),
[(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO 2006/129714),
2-Cyan-3-(difluormethoxy)-N,N-dimethylbenzolsulfonamid (bekannt aus WO2006/056433),
2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407) und N-[1-(2,3-Dimethylphenyl)-2-(3,5-dimethylphenyl)ethyl]-4,5-dihydro-1,3-thiazol-2-amin (bekannt aus WO2008/104503).

In einer bevorzugten Ausführungsform der Erfindung wird den Pflanzenschutzmitteln zur Wirkungssteigerung zusätzlich ein Penetrationsförderer zugegeben. Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester, insbesondere Rapsölmethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Beispielhaft seien die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) genannt. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf (Kartoffel) vertrieben werden. Als Beispiele für Herbizid-tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp. (Ctenocephalides canis, Ctenocephalides felis), Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele

### Beispiel E: 5-(Pyridin-3-yl)-N-(2,2,2-trifluorethyl)-furan-3-carboxamid

### Stufe 1: 5-(Pyridin-3-yl)-furan-3-carbonsäure

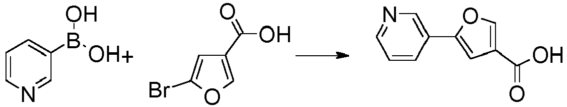

31 mg (0,17 mmol) Palladium(II)chlorid und 250 mg (0,95 mmol) Triphenylphosphan wurden mit 0,5 g Tetrabutylammoniumchlorid in 50 ml DMF unter Argon 5 min bei 95°C gerührt, dann mit einer Lösung in DMF von 6,20 g (50,4 mmol) Pyridin-3-boronsäure (erhalten nach Tetrahedron Lett. 2002, 4285 bzw. J. Org. Chem. 2002, 5394) und 8,20 g (42,9 mmol) 5-Brom-furan-3-carbonsäure (verunreinigt, erhalten nach Bull. Chem. Soc. France 1971, 990) sowie 16,0 g (114 mmol) Kaliumcarbonat als Lösung in 30 ml Wasser versetzt und 16 Stunden bei 95°C unter Argon gerührt. Man versetzte mit wässriger Zitronensäure, engte ein, löste in verdünnter Natronlauge, wässriger Natriumchlorid, *n*-Butanol, versetzte mit Natriumchlorid bis zur Sättigung, extrahiert dreimal mit *n*-Butanol und dampfte die vereinigten organischen Phasen ein. Man versetzte mit 400 ml Methanol, rührte bei 40°C, filtrierte, engte bis zur Trübung ein, saugte vom Niederschlag ab und wiederholte die Prozedur mit dem Filtrat. Die vereinigten Filterkuchen wurden am Rotationsverdampfer getrocknet und durch Chromatographie an Kieselgel (Laufmittel: Aceton / MTBE 30% → Aceton / Methanol 20%) gereinigt.

### Ausbeute 1,68 g (15% d. Th.); HPLC-MS: logP (HCOOH) = 0,07; Masse (m/z): 190,1 (M+H)⁺;

¹H-NMR (d₆-DMSO): 7,30 (s, 1H), 7,45 (m, 1H), 8,10 (d, 1H), 8,30 (s, 1H), 8,50 (m, 1H), 8,95 ppm (s, 1H).

### Stufe 2 : 5-(Pyridin-3-yl)-N-(2,2,2-trifluorethyl)-furan-3-carboxamid

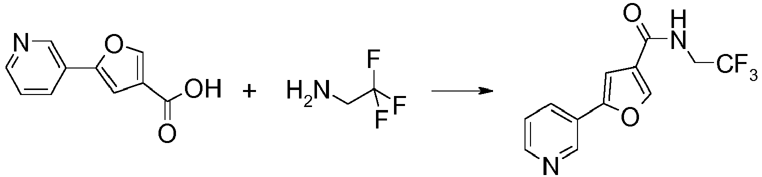

245 mg (1,30 mmol) 5-(Pyridin-3-yl)-furan-3-carbonsäure wurden in Acetonitril mit 3,00 ml (17 mmol) *N,N*-Diisopropylethylamin gelöst und mit 0,52 g (2 mmol) BOP-Cl versetzt. Nach 20 Minuten versetzte man mit 0,30 ml (3,90 mmol) 2,2,2-Trifluorethylamin, die Mischung wurde 16 Stunden gerührt, dann eingeengt, mit Ethylacetat, wäßrigem Natriumchlorid und Phosphatpuffer pH 7 versetzt und zweimal mit Ethylacetat extrahiert. Die vereinigten organischen Phasen wurden mit Na₂SO₄ getrocknet und eingeengt. Der Rückstand wurde durch Chromatographie an Kieselgel (Laufmittel: Cyclohexan / Aceton) gereinigt.

### Ausbeute: 30,0 mg (7% d. Th.); HPLC-MS: logP (HCOOH) = 1,13; Masse (m/z): 271,0 (M+H)⁺;

¹H-NMR (CD₃CN): 4,05 (m, 2H), 7,20 (s, 1H), 7,25 (br, 1H), 7,40 (m, 1H), 8,00 (m, 1H), 8,15 (s, 1H), 8,55 (m, 1H), 8,95 ppm (s, 1H).

### Beispiel G: N-Methyl-5-(pyridin-3-yl)-furan-2-carboxamid

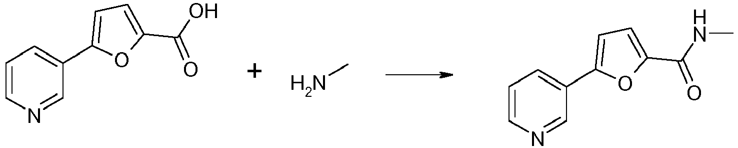

5-(Pyridin-3-yl)-furan-2-carbonsäure (200 mg, 1,06 mmol) (Herstellung siehe US 3,927,008) wurde in 30 ml Dioxan vorgelegt und mit *N*-(3-Dimethylaminopropyl)-*N'*-ethyl-carbodiimid hydrochlorid (253 mg, 1,32 mmol) versetzt. Unter Rühren wurde eine 2 M Lösung von Methylamin in Tetrahydrofuran (41,0 mg, 1,32 mmol) zugetropft. Man rührte 3 Stunden und setzte dann 1 ml Wasser zu. Die Reaktionsmischung wurde über Nacht bei Raumtemperatur gerührt und das Lösungsmittel anschließend unter vermindertem Druck entfernt. Der Rückstand wurde in Wasser aufgenommen und mit Ethylacetat extrahiert. Die organische Phase wurde über Magnesiumsulfat getrocknet, das Lösungsmittel am Rotationsverdampfer entfernt und der Rückstand über Kieselgel (Laufmittel: Cyclohexan, Ethylacetat, 1:1 Gemisch) chromatographiert.

### Ausbeute: 171 mg (75% d. Th.); HPLC-MS: logP (HCOOH) = 0,42; Masse (m/z): 203,1 (M+H)⁺;

¹H-NMR (d₆-DMSO): 2,56 (d, 3H), 7,50-7,55 (m, 2H), 7,66 (m, 1H), 8,30 (m, 1H), 8,80 (m, 1H), 9,23 (m,1H), 9,32 ppm (m, 1H).

### Beispiel J: 1-(Pyridin-3-yl)-N-(2,2,2-trifluorethyl)-pyrrol-3-carboxamid

### Stufe 1: 1-(Pyridin-3-yl)-pyrrol-3-carbonsäuremethylester

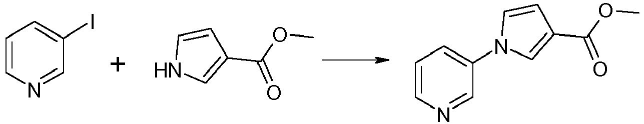

Unter Argon wurden 250 mg (1,18 mmol) 3-Iodpyridin mit 123 mg (0,98 mmol) 1*H*-Pyrrol-3-carbonsäuremethylester, 28,1 mg (0,14 mmol) Cu(I)iodid, 21.5 mg (0,14 mmol) 8-Hydroxychinolin und 150 mg (1,08 mmol) Kaliumcarbonat in 1 ml Dimethylformamid versetzt und im Mikrowellenreaktor zur Reaktion gebracht. Nach 40 Minuten (200 Watt, 150°C) wurde das Reaktionsgemisch abgekühlt und über eine 1 g Kieselgelkartusche filtriert, die Kartusche nochmals mit Dimethylformamid gewaschen, die Lösungen vereinigt und im Vakuum eingeengt. Der Rückstand wurde mit Essigester/Wasser versetzt und ausgeschüttelt. Die organische Phase wurde abgetrennt und über Natriumsulfat getrocknet. Nach Eindampfen der Lösung im Vakuum wurde das Produkt erhalten.

Ausbeute: 146 mg (85% Reinheit, 52% d. Th.); HPLC-MS: logP (HCOOH) = 1,26; Masse (m/z): 203,0 (M+H)⁺.

### Stufe 2: Lithiumsalz der 1-(Pyridin-3-yl)-pyrrol-3-carbonsäure

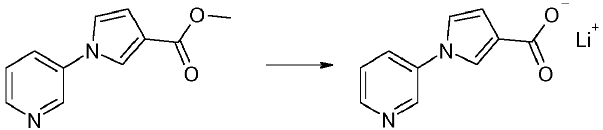

147 mg (720 µmol) 1-(Pyridin-3-yl)-pyrrol-3-carbonsäuremethylester wurden in 1 ml Tetrahydrofuran vorgelegt und mit einer Lösung von 88,8 mg (3,62 mmol) Lithiumhydroxid in wenig Methanol/Wasser (1:1) versetzt. Die nach Zugabe der Lithiumhydroxid-Lösung zunächst gebildete Suspension ging klar in Lösung. Nach 48 Stunden bei Raumtemperatur wurde 15 Stunden bei 50°C gerührt. Nach dem Einengen im Vakuum wurde das rohe Lithiumsalz erhalten, das ohne weitere Reinigung im nächsten Reaktionsschritt eingesetzt wurde.

### Ausbeute: 149 mg (100% d. Th.)

### Stufe 3: 1-(Pyridin-3-yl)-N-(2,2,2-trifluorethyl)-pyrrol-3-carboxamid

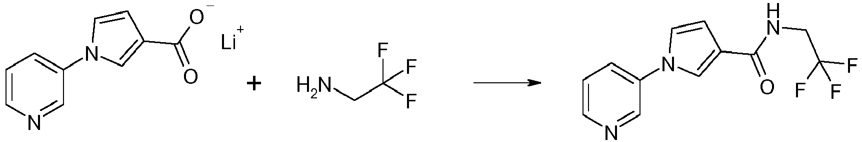

149 mg (760 µmol) Lithiumsalz der 1-(Pyridin-3-yl)-pyrrol-3-carbonsäure wurden in 3 ml *N*,*N-*Dimethylformamid gelöst und 10 Minuten bei Raumtemperatur gerührt. Nach Zugabe von 82,0 mg (8,30 mmol) Trifluorethylamin und einer Lösung von *N,N-*Diisopropylethylamin in Dimethylformamid wurde 15 Stunden bei Raumtemperatur gerührt. Nach Eindampfen der Reaktionslösung, wurde der verbleibende Rückstand mittels Mitteldruckchromatographie gereinigt.

### Ausbeute: 101 mg (10% d. Th.); HPLC-MS: logP (HCOOH) = 1,24; Masse (m/z): 270,0 (M+H)⁺;

¹H-NMR (d₆-DMSO): 4,05 (m, 2H), 6,81 (m, 1H), 7,50-7,55 (m, 2H), 8,07 (m, 2H), 8,50 (m, 2H), 8,90 ppm (m, 1H).

Analog bzw. gemäß den allgemeinen Angaben zur Herstellung wurden die in der folgenden Tabelle aufgeführten Verbindungen hergestellt:

| **Verbindungsnummer** | **Struktur** | **logP¹ (HCO₂H)** | **(M+H)⁺** | **NMR Daten²** |
|---|---|---|---|---|
| 6 | 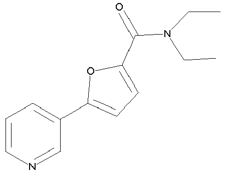 | 1,23 | 245,1 | ¹H-NMR (CD₃CN): 1,30 (br, 6H), 3,60 (br, 4H), 6,95 (d, 1H), 7,05 (d, 1H), 7,40 (m, 1H), 8,05 (m, 1H), 8,55 (m, 1H), 8,95 ppm (s, 1H). |
| 7 | 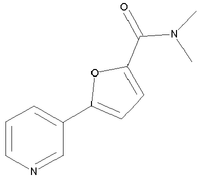 | 0,51 | 217,1 | ¹H-NMR (CD₃CN): 3,00-3,40 (br, 6H), 6,95 (d, 1H), 7,05 (d, 1H), 7,40 (m, 1H), 8,05 (m, 1H), 8,55 (m, 1H), 8,95 ppm (m, 1H). |
| 8 | 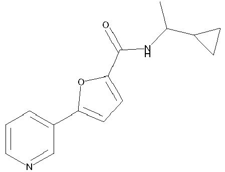 | 1,36 | 257,0 | ¹H-NMR (CD₃CN): 0,25 (m, 1H), 0,35 (m, 1H), 0,45 (m, 1H), 0,55 (m, 1H), 1,05 (m, 1H), 1,30 (m, 3H), 3,45 (m, 1H), 6,95 (m, 1H), 7,10 (m, 2H), 7,40 (m, 1H), 8,15 (m, 1H), 8,55 (m, 1H), 9,05 ppm (m 1H). |
| 9 | 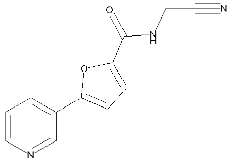 | 0,43 | 228,1 | ¹H-NMR (CD₃CN):4,25 (m, 2H), 7,00 (d, 1H), 7,20 (d, 1H), 7,45 (m, 1H), 7,70 (br, 1H), 8,15 (d, 1H), 8,55 (m, 1H), 9,05 ppm (s, 1H). |
| 10 | 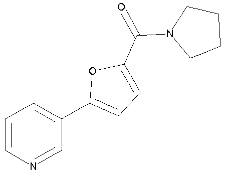 | 1,06 | 243,2 | ¹H-NMR (CD₃CN): 1,90 (m, 4H), 3,55(m, 2H), 3,90 (m, 2H), 7,00 (d, 1H), 7,01 (d, 1H), 7,40 (m, 1H), 8,05 (m, 1H), 8,55 (m, 1H), 9,00 ppm (s, 1H). |
| 11 | 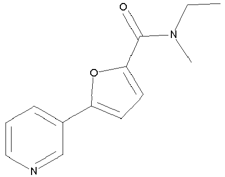 | 1,00 | 231,1 | ¹H-NMR (CD₃CN): 1,20 (br, 3H), 3,10 (br, 3H), 3,60 (br, 2H), 6,95 (d, 1H), 7,05 (d, 1H), 7,40 (m, 1H), 8,05 (m, 1H), 8,55 (m, 1H), 8,95 ppm (s, 1H). |
| 12 (Beispiel E) | 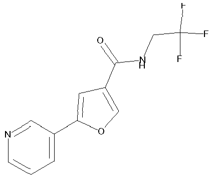 | 1,13 | 271,0 | ¹H-NMR (CD₃CN): 4,05 (m, 2H), 7,20 (s, 1H), 7,25 (br, 1H), 7,40 (m, 1H), 8,00 (m, 1H), 8,15 (s, 1H), 8,55 (m, 1H), 8,95 ppm (s, 1H). |
| 15 | 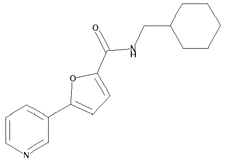 | 2,02 | 285,2 | ¹H-NMR (CD₃CN): 1,00-1,90 (m, 11H), 3,20 (m, 2H), 6,90 (d, 1H), 7,10 (d, 1H), 7,40 (m, 1H), 8,10 (m, 1H), 8,50 (m, 1H), 9,00 ppm (s, 1H). |
| 16 | 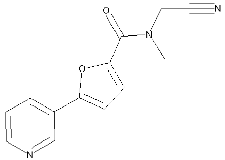 | 0,72 | 242,1 | ¹H-NMR (CD₃CN): 3,35 (s, 3H), 4,50 (s, 2H), 7,00 (d, 1H), 7,20 (d, 1H), 7,40 (m, 1H), 8,05 (m, 1H), 8,55 (m, 1H), 9,00 ppm (s, 1H). |
| 17 | 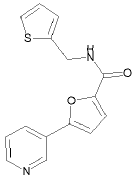 | 1,46 | 285,1 | ¹H-NMR (CD₃CN): 4,70 (m, 2H), 7,00 (m, 2H), 7,05 (m, 1H), 7,20 (m, 1H), 7,30 (m, 1H), 7,40 (m, 1H), 7,80 (br, 1H), 8,10 (m, 1H), 8,50 (m, 1H), 9,00 ppm (s, 1H). |
| 18 | 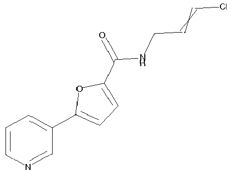 | 1,27; 1,33 (Isomerengemisch) | 263,1 | ¹H-NMR (CD₃CN): 3,95 (m, 1H), 4,15 (m, 1H), 5,90-6,10 (m, 1H), 6,20-6,30 (m, 1H), 7,00 (d, 1H), 7,10 (d, 1H), 7,40 (m, 2H), 8,10 (m, 1H), 8,50 (m, 1H), 9,00 ppm (s, 1H) |
| 19 | 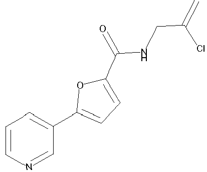 | 1,28 | 263,1 | ¹H-NMR (CD₃CN): 4,10 (m, 2H), 5,35 (s, 1H), 5,50 (m, 1H), 7,00 (d, 1H), 7,10 (d, 1H), 7,45 (m, 1H), 7,60 (br, 1H), 8,15 (m, 1H), 8,55 (m, 1H), 9,05 ppm (s, 1H). |
| 20 | 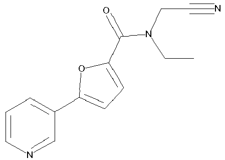 | 1,11 | 256,0 | ¹H-NMR (CD₃CN): 1,35 (t, 3H), 3,80 (br, 2H), 4,40 (br, 2H), 7,00 (d, 1H), 7,20 (d, 1H), 7,45 (m, 1H), 8,10 (d, 1H), 8,6 (m, 1H), 9,00 ppm (s, 1H). |
| 21 | 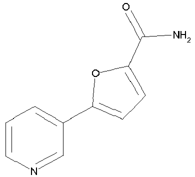 | - | 189,1 | ¹H-NMR (CD₃CN): 6,00 (br, 1H), 6,90 (br, 1H), 7,00 (d, 1H), 7,10 (d, 1H), 7,40 (m, 1H), 8,10 (m, 1H), 8,55 (m, 1H), 9,00 ppm (s, 1H). |
| 22 | 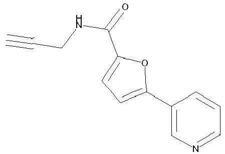 | 0,72 | 227,1 | ¹H-NMR (CD₃CN): 2,40 (s, 1H), 4,10 (m, 2H), 7,00 (d, 1H), 7,15 (d, 1H), 7,40 (m, 2H), 8,10 (m, 1H), 8,55 (m, 1H), 9,00 ppm (s, 1H). |
| 23 | 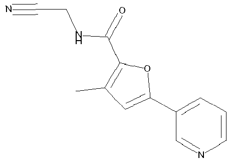 | 1,07 | 242,1 | ¹H-NMR (CD₃CN): 2,40 (s, 3H), 4,20 (m, 2H), 6,85 (s, 1H), 7,40 (m, 1H), 7,50 (br, 1H), 8,10 (d, 1H), 8,55 (m, 1H), 9,00 ppm (s, 1H). |
| 24 | 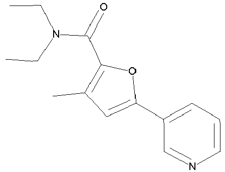 | 1,58 | 259,2 | ¹H-NMR (CD₃CN): 1,25 (t, 6H), 2,25 (s, 3H), 3,50 (q, 4H), 6,80 (s, 1H), 7,35 (m, 1H), 8,00 (d, 1H), 8,50 (m, 1H), 8,90 ppm (s, 1H). |
| 26 | 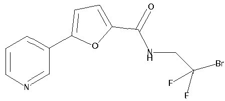 | 1,49 | 332,9 | ¹H-NMR (CD₃CN): 4,25 (m, 2H), 7,00 (d, 1H), 7,20 (d, 1H), 7,40 (m, 1H), 7,60 (br, 1H), 8,10 (d, 1H), 8,55 (m, 1H), 9,05 ppm (s, 1H). |
| 27 | 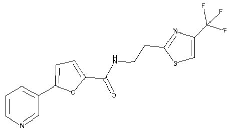 | 1,69 | 368,0 | ¹H-NMR (CD₃CN): 3,35 (m, 2H), 3,80 (m, 2H), 7,00 (d, 1H), 7,10 (d, 1H), 7,40 (m, 2H), 7,90 (m, 1H), 8,15 (m, 1H), 8,50 (m, 1H), 9,00 ppm (s, 1H). |
| 30 | 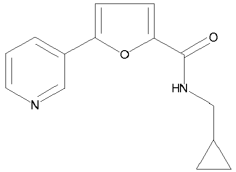 | 1,39 | 243,2 | ¹H-NMR (d₆-DMSO): 0,50 (m, 2H), 0,80 (m, 2H), 1,00 (m, 1H), 3,13 (m, 2H), 7,50-7,55 (m,2H), 7,65 (m, 1H), 8,32 (m, 1H), 8,75 (m, 1H), 9,20 (m, 1H), 9,33 ppm (m, 1H) |
| 31 (Beispiel G) | 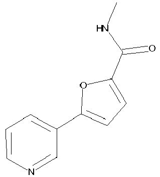 | 0,42 | 203,1 | ¹H-NMR (d₆-DMSO): 2,56 (d, 3H), 7,50-7,55 (m, 2H), 7,66 (m, 1H), 8,30 (m, 1H), 8,80 (m, 1H), 9,23 (m, 1H), 9,32 ppm (m, 1H). |
| 32 | 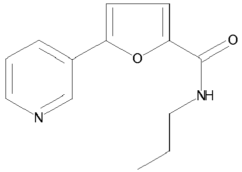 | 0,83 | 231,1 | - |
| 36 (Beispiel J) | 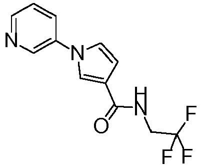 | 1,24 | 270,0 | ¹H-NMR (d₆-DMSO): 4,05 (m, 2H), 6,81 (m, 1H), 7,50-7,55 (m, 2H), 8,07 (m, 2H), 8,50 (m, 2H), 8,90 ppm (m, 1H). |

### 1 Methodenbeschreibung zur Bestimmung der logP-Werte und Massendetektion

Die Bestimmung der in der Tabelle angegebenen logP-Werte erfolgte gemäß EEC-Directive 79/831 Annex V.A8 durch HPLC (High Performance Liquid Chromato-graphy) an einer Phasenumkehrsäule (C 18). Temperatur: 55°C.

Eluenten für die Bestimmung im sauren Bereich (pH 3,4):
Eluenat A: Acetonitril + 1 ml Ameisensäure/L. Eluent B: Wasser + 0,9 ml Ameisensäure/L.
Gradient: von 10% Eluent A / 90% Eluent B bis 95% Eluent A / 5% Eluent B in 4,25 min.

Die Eichung erfolgte mit unverzweigten Alkan-2-onen (mit 3 bis 16 Kohlen-stoff-atomen), deren logP-Werte bekannt sind (Bestimmung der logP-Werte anhand der Retentionszeiten durch lineare Interpolation zwischen zwei aufeinander folgenden Alkanonen). Die λₘₐₓ-Werte wurden an Hand der UV-Spektren von 200 nm bis 400 nm in den Maxima der chromatographischen Signale ermittelt. Die Massendetektion (M⁺, m/z) erfolgte über ein Agilent MSD Massensprektrometer.

### 2 Messung der NMR-Spektren

Die NMR-Spektren wurden mit einem Bruker Avance 400, ausgestattet mit einem Durchflussprobenkopf (60 µl Volumen), bestimmt. Als Lösungsmittel wurden CD₃CN, CDCl₃ oder d₆-DMSO verwendet, wobei als Referenz Tetramethylsilan (0,00 ppm) eingesetzt wurde. In Einzelfällen wurden die NMR Spektren mit einem Bruker Avance II 600 oder einem Varian Unity Inova 400 bestimmt.

Die Aufstpaltung der Signale wurde wie folgt beschrieben: s (Singulett), d (Duplett), t (Triplett), q (Quartett), quin (Quintett), m (Multiplett), br (breit).

### Biologische Beispiele

**Myzus persicae - Test (Spritzbehandlung)**

| | |
|---|---|
| Lösungsmittel: | 78 GewichtsteileAceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis),* die von allen Stadien der Grünen Pfirsichblattlaus (*Myzus persicae)* befallen sind, werden mit einer Wirkstoffzubereitung der gewünschten Konzentration gespritzt.

Nach 6 Tagen wird die Wirkung in % bestimmt. Dabei bedeutet 100%, dass alle Blattläuse abgetötet wurden; 0% bedeutet, dass keine Blattläuse abgetötet wurden.

Bei diesem Test zeigten z. B. die folgenden Verbindungen der Herstellungsbeispiele Wirkung von 100% bei einer Aufwandmenge von 500g/ha: 6, 7, 9, 11, 17, 19, 20, 22, 23, 30, 31, 32.

Bei diesem Test zeigten z. B. die folgenden Verbindungen der Herstellungsbeispiele eine Wirkung von 90% bei einer Aufwandmenge von 500g/ha: 12, 16, 18, 24, 36.

Bei diesem Test zeigten z. B. die folgenden Verbindungen der Herstellungsbeispiele eine Wirkung von 80% bei einer Aufwandmenge von 500g/ha: 8, 27.

## Patentansprüche

1. Verwendung von Verbindungen der Formel (I) 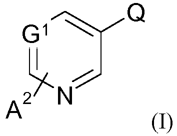 worin
G¹ für C-A¹ steht,
A¹ für Wasserstoff steht,
A² für Wasserstoff steht,
Q für einen der folgenden Reste 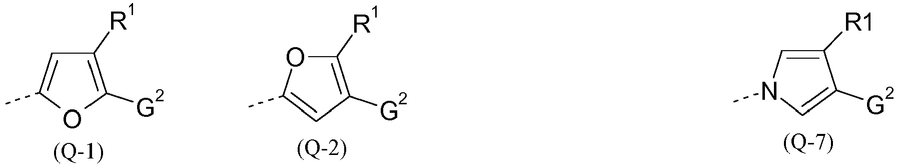
steht,
R¹ für Wasserstoff, C₁-C₆-Alkyl, C₃-C₆-Cycloalkyl, Halogen, Cyano, C₁-C₃-Haloalkyl, Hydroxy oder C₁-C₆-Alkoxy steht,
G² für C(=X)NR³R⁴ steht,
X für Sauerstoff oder Schwefel steht,
R³ für einen Rest aus der Reihe Wasserstoff, C₁-C₆-Alkyl, C₃-C₆-Cycloalkyl, C₁-C₆-Halogenalkyl, Cyano-C₁-C₆-alkyl, C₁-C₆-Alkoxy, C₁-C₆-Halogenalkoxy, C₂-C₆-Alkenyl, C₁-C₆-Alkoxy-C₁-C₆-alkyl, gegebenenfalls durch Halogen substituiertes C₁-C₆-Alkylcarbonyl, gegebenenfalls durch Halogen substituiertes C₁-C₆-Alkoxycarbonyl, gegebenenfalls durch Halogen, C₁-C₆-Alkyl, C₁-C₆-Alkoxy, C₁-C₆-Halogenalkyl und Cyano substituiertes C₃-C₆-Cycloalkylcarbonyl, oder für ein Kation, wie beispielsweise ein ein- oder zweiwertiges Metallion oder ein gegebenenfalls durch C₁-C₆-Alkyl oder Aryl-C₁-C₆-alkyl substituiertes Ammonium-Ion steht,
R⁴ für einen Rest aus der Reihe Wasserstoff, Cyano, jeweils gegebenenfalls durch Halogen, Cyano, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl und C₁-C₆-Halogenalkylsulfonyl substituiertes C₁-C₆-Alkyl, C₂-C₆-Alkenyl und C₂-C₆-Alkinyl, C₁-C₆-Alkoxy, C₁-C₆-Halogenalkoxy, gegebenenfalls durch Halogen substituiertes Bis(C₁-C₆-alkoxy)-C₁-C₆-alkyl, gegebenenfalls durch Halogen substituiertes Bis(C₁-C₆-alkylthio)-C₁-C₆-alkyl, gegebenenfalls durch Halogen substituiertes C₁-C₆-Alkylcarbonyl-C₁-C₆-alkyl, gegebenenfalls durch Halogen substituiertes C₁-C₆-Alkylsulfinyl-C₁-C₆-alkyl, gegebenenfalls durch Halogen substituiertes C₁-C₆-Alkylsulfonyl-C₁-C₆-alkyl, gegebenenfalls durch Halogen substituiertes C₁-C₆-Alkoxycarbonyl-C₁-C₆-alkyl, C₂-C₄-Alkinyloxy, gegebenenfalls durch Halogen substituiertes C₃-C₆-Cycloalkylcarbonyl, gegebenenfalls durch Halogen substituiertes C₃-C₆-Cycloalkyl-C₁-C₆-alkyl, jeweils gegebenenfalls durch Halogen, C₁-C₆-Alkyl, C₁-C₆-Halogenalkyl, C₁-C₆-Alkoxy oder C₁-C₆-Halogenalkoxy substituiertes C₃-C₆-Cycloalkyl und C₃-C₆-Cycloalkenyl, in welchen die Ringe ein bis drei Heteroatome aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, Cs-C₆-Cycloalkyl-C₁-C₆-alkyl, C₁-C₆-Alkylcarbonyl, C₁-C₆-Alkoxycarbonyl oder Aminocarbonyl substituiertes Aryl, Heteroaryl, Heterocyclyl, Heterocyclyl-C₁-C₆-alkyl, Aryl-C₁-C₆-alkyl, Heteroaryl-C₁-C₆-alkyl, oder für NR⁵R⁶ steht, worin R⁵ und R⁶ unabhängig voneinander für einen Rest aus der Reihe Wasserstoff, C₁-C₆-Alkyl, C₁-C₆-Haloalkyl, C₃-C₆-Cycloalkyl, C₁-C₆-Alkoxy, C₁-C₆-Alkylcarbonyl, C₁-C₆-Alkoxylcarbonyl, Hetaryl und Heterocyclyl stehen oder R⁵ und R⁶ gemeinsam mit dem Stickstoffatom, an das sie gebunden sind, einen gegebenenfalls durch Halogen substituierten Heterocyclus bilden,
R³ und R⁴ auch gemeinsam mit dem Stickstoffatom, an das sie gebunden sind, einen gegebenenfalls durch C₁-C₆-Alkyl, C₁-C₆-Alkoxy oder Halogen substituierten und gegebenenfalls ein oder zwei Heteroatome aus der Reihe Sauerstoff, Stickstoff und Schwefel enthaltenden 3- bis 7-gliedrigen Ring bilden können, wobei Sauerstoffatome nicht unmittelbar benachbart sein dürfen,
sowie Salzen, N-Oxiden und tautomeren Formen der Verbindungen der Formel (I), wobei Halogen ausgewählt ist aus der Reihe Fluor, Chlor, Brom und Iod,
Aryl (auch als Teil einer größeren Einheit, wie beispielsweise Arylalkyl) ausgewählt ist aus der Reihe Phenyl, Naphthyl, Anthryl, Phenanthrenyl und
Hetaryl (gleichbedeutend mit Heteroaryl, auch als Teil einer größeren Einheit, wie beispielsweise Hetarylalkyl) ausgewählt ist aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
zur Bekämpfung von tierischen Schädlingen.

2. Verbindungen der Formel (I) 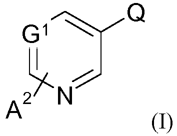 worin
G¹ für C-A¹ steht,
A¹ für Wasserstoff steht,
A² für Wasserstoff steht,
Q für einen der folgenden Reste 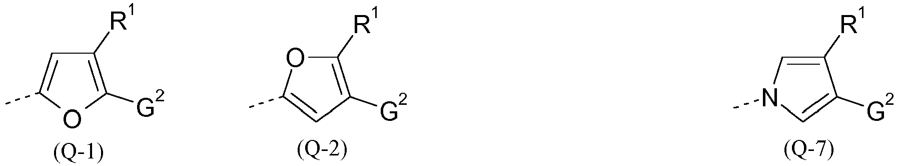
R¹ für Wasserstoff, C₁-C₄-Alkyl, C₃-C₆-Cycloalkyl, Halogen, Cyano, C₁-C₃-Haloalkyl, Hydroxy oder C₁-C₄-Alkoxy steht,
G² für C(=X)NR³R⁴ steht,
X und Y unabhängig voneinander für Sauerstoff oder Schwefel stehen,
R³ für einen Rest aus der Reihe Wasserstoff, C₁-C₄-Alkyl, C₃-C₆-Cycloalkyl, C₁-C₄-Halogenalkyl, Cyano-C₁-C₄-alkyl, C₁-C₄-Alkoxy, C₁-C₄-Halogenalkoxy, C₂-C₄-Alkenyl, C₁-C₄-Alkoxy-C₁-C₄-alkyl, gegebenenfalls durch Halogen substituiertes C₁-C₄-Alkylcarbonyl, gegebenenfalls durch Halogen substituiertes C₁-C₄-Alkoxycarbonyl, gegebenenfalls durch Halogen, C₁-C₄-Alkyl, C₁-C₄-Alkoxy, C₁-C₄-Halogenalkyl und Cyano substituiertes C₃-C₆-Cycloalkylcarbonyl, oder für ein Kation, wie beispielsweise ein ein- oder zweiwertiges Metallion oder ein gegebenenfalls durch C₁-C₄-Alkyl oder Aryl-C₁-C₄-alkyl substituiertes Ammonium-Ion steht,
R⁴ für einen Rest aus der Reihe Wasserstoff, Cyano, jeweils gegebenenfalls durch Halogen, Cyano, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl und C₁-C₄-Halogenalkylsulfonyl substituiertes C₁-C₄-Alkyl, C₂-C₄-Alkenyl und C₂-C₄-Alkinyl, C₁-C₄-Alkoxy, C₁-C₄-Halogenalkoxy, gegebenenfalls durch Halogen substituiertes Bis(C₁-C₄-alkoxy)-C₁-C₄-alkyl, gegebenenfalls durch Halogen substituiertes Bis(C₁-C₄-alkylthio)-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, gegebenenfalls durch Halogen substituiertes C₁-C₄-Alkoxycarbonyl-C₁-C₄-alkyl, C₂-C₄-Alkinyloxy, gegebenenfalls durch Halogen substituiertes C₃-C₆-Cycloalkyl-carbonyl, gegebenenfalls durch Halogen substituiertes C₃-C₆-Cycloalkyl-C₁-C₄-alkyl, jeweils gegebenenfalls durch Halogen, C₁-C₄-Alkyl, C₁-C₄-Halogenalkyl, C₁-C₄-Alkoxy oder C₁-C₄-Halogenalkoxy substituiertes C₃-C₆-Cycloalkyl und C₃-C₆-Cycloalkenyl, in welchen die Ringe ein bis drei Heteroatome aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₄-Alkyl, C₁-C₄-Halogenalkyl,C₃-C₆-Cycloalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Aryl, Heteroaryl, Aryl-C₁-C₄-alkyl, Heteroaryl-C₁-C₄-alkyl, oder für NR⁵R⁶ steht, worin R⁵ und R⁶ unabhängig voneinander für einen Rest aus der Reihe Wasserstoff, C₁-C₄-Alkyl, C₁-C₄-Haloalkyl, C₃-C₆-Cycloalkyl, C₁-C₄-Alkoxy, C₁-C₄-Alkylcarbonyl, C₁-C₄-Alkoxylcarbonyl, Hetaryl und Heterocyclyl stehen oder R⁵ und R⁶ gemeinsam mit dem Stickstoffatom, an das sie gebunden sind, einen gegebenenfalls durch Halogen substituierten Heterocyclus bilden,
R³ und R⁴ auch gemeinsam mit dem Stickstoffatom, an das sie gebunden sind, einen gegebenenfalls durch C₁-C₄-Alkyl, C₁-C₄-Alkoxy oder Halogen substituierten und gegebenenfalls ein oder zwei Heteroatome aus der Reihe Sauerstoff, Stickstoff und Schwefel enthaltenden 3- bis 7-gliedrigen Ring bilden können, wobei Sauerstoffatome nicht unmittelbar benachbart sein dürfen,
und wobei
Halogen ausgewählt ist aus der Reihe Fluor, Chlor, Brom und Iod,
Aryl (auch als Teil einer größeren Einheit, wie beispielsweise Arylalkyl) ausgewählt ist aus der Reihe Phenyl, Naphthyl, Anthryl, Phenanthrenyl und
Hetaryl (auch als Teil einer größeren Einheit, wie beispielsweise Hetarylalkyl) ausgewählt ist aus der Reihe Thienyl, Pyrimidyl, Oxadiazolyl, Oxazolyl, Pyrazinyl, Imidazolyl, Thiazolyl und Furanyl.

3. Verbindungen der Formel (I) gemäß Anspruch 2, worin
G¹ für C-A¹ steht,
A¹ für Wasserstoff steht,
A² für Wasserstoff steht,
Q für Q-1, Q-2 oder Q-7 steht,
R¹ für Wasserstoff oder C₁-C₄-Alkyl steht,
G² für C(=X)NR³R⁴ steht,
X für Sauerstoff steht,
R³ für einen Rest aus der Reihe Wasserstoff und C₁-C₄-Alkyl steht,
R⁴ für einen Rest aus der Reihe Wasserstoff, jeweils gegebenenfalls durch Halogen, Cyano und C₁-C₄-Alkylthio substituiertes C₁-C₄-Alkyl und C₂-C₄-Alkenyl, für C₂-C₄-Alkinyl, gegebenenfalls durch Halogen substituiertes C₃-C₆-Cycloalkyl-C₁-C₄-alkyl und gegebenenfalls durch C₁-C₄-Halogenalkyl substituiertes Heteroaryl-C₁-C₄-alkyl steht und
R³ und R⁴ auch gemeinsam mit dem Stickstoffatom, an das sie gebunden sind, einen 3- bis 7-gliedrigen Ring bilden können, der keine weiteren Heteroatome enthält und wobei
Halogen ausgewählt ist aus der Reihe Fluor, Chlor, Brom und Iod und
Hetaryl (auch als Teil einer größeren Einheit, wie beispielsweise Hetarylalkyl) ausgewählt ist aus der Reihe Thienyl, Pyrimidyl und Thiazolyl.

4. Verfahren zum Bekämpfen von tierischen Schädlingen **dadurch gekennzeichnet, dass** man eine Verbindung der Formel (I) gemäß Anspruch 2 oder 3 auf die Schädlinge und/oder ihren Lebensraum einwirken lässt, wobei Verfahren am tierischen oder menschlichen Körper ausgenommen sind.

## Claims

1. Use of compounds of the formula (I) 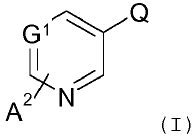 in which
G¹ is C-A¹,
A¹ is hydrogen,
A² is hydrogen,
Q is one of the following radicals 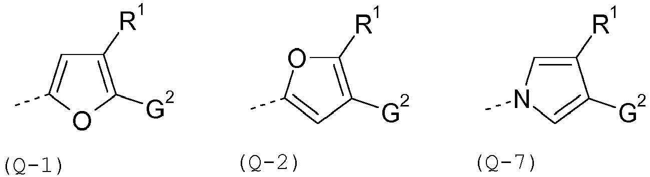
R¹ is hydrogen, C₁-C₆-alkyl, C₃-C₆-cycloalkyl, halogen, cyano, C₁-C₃-haloalkyl, hydroxyl or C₁-C₆-alkoxy,
G² is C (=X) NR³R⁴,
X is oxygen or sulphur,
R³ is a radical from the group of hydrogen, C₁-C₆-alkyl, C₃-C₆-cycloalkyl, C₁-C₆- haloalkyl, cyano-C₁-C₆-alkyl, C₁-C₆-alkoxy, C₁-C₆- haloalkoxy, C₂-C₆-alkenyl, C₁-C₆-alkoxy-C₁-C₆-alkyl, optionally halogen-substituted C₁-C₆-alkylcarbonyl, optionally halogen-substituted C₁-C₆-alkoxycarbonyl, optionally halogen-, C₁-C₆-alkyl-, C₁-C₆-alkoxy-, C₁-C₆-haloalkyl- and cyano-substituted C₃-C₆-cycloalkylcarbonyl, or a cation, for example a mono- or divalent metal ion or an optionally C₁-C₆-alkyl- or aryl-C₁-C₆-alkyl-substituted ammonium ion,
R⁴ is a radical from the group of hydrogen, cyano, in each case optionally halogen-, cyano-, C₁-C₆-alkoxy-, C₁-C₆-haloalkoxy-, C₁-C₆-alkyl thio-, C₁-C₆-haloalkyl thio-, C₁-C₆-alkyl sulphinyl-, C₁-C₆-haloalkylsulphinyl-, C₁-C₆-alkylsulphonyl- and C₁-C₆-haloalkylsulphonyl-substituted C₁-C₆-alkyl, C₂-C₆-alkenyl and C₂-C₆-alkynyl, C₁-C₆-alkoxy, C₁-C₆-haloalkoxy, optionally halogen-substituted bis(C₁-C₆-alkoxy)-C₁-C₆-alkyl, optionally halogen-substituted bis(C₁-C₆-alkylthio)-C₁-C₆-alkyl, optionally halogen-substituted C₁-C₆-alkylcarbonyl-C₁-C₆-alkyl, optionally halogen-substituted C₁-C₆-alkylsulphinyl-C₁-C₆-alkyl, optionally halogen-substituted C₁-C₆-alkylsulphonyl-C₁-C₆-alkyl, optionally halogen-substituted C₁-C₆-alkoxycarbonyl-C₁-C₆-alkyl, C₂-C₄-alkynyloxy, optionally halogen-substituted C₃-C₆-cycloalkylcarbonyl, optionally halogen-substituted C₃-C₆-cycloalkyl-C₁-C₆-alkyl, in each case optionally halogen-, C₁-C₆-alkyl-, C₁-C₆-haloalkyl-, C₁-C₆-alkoxy- or C₁-C₆-haloalkoxy-substituted C₃-C₆-cycloalkyl and C₃-C₆-cycloalkenyl, in which the rings may contain one to three heteroatoms from the group of sulphur, oxygen (where oxygen atoms must not be immediately adjacent) and nitrogen, in each case optionally halogen-, cyano- (including in the alkyl moiety), nitro-, C₁-C₆-alkyl-, C₁-C₆-haloalkyl-, C₃-C₆-cycloalkyl-, C₁-C₆-alkoxy-, C₁-C₆-haloalkoxy-, C₁-C₆-alkylthio-, C₁-C₆- haloalkyl thio-, C₁-C₆-alkylsulphinyl-, C₁-C₆-haloalkylsulphinyl-, C₁-C₆-alkylsulphonyl-, C₁-C₆-haloalkyl sulphonyl-, amino-, C₁-C₆-alkylamino-, di (C₁-C₆-alkyl) amino-, C₁-C₆-alkylcarbonylamino-, C₁-C₆-alkoxycarbonylamino-, C₁-C₆-alkoxy-C₁-C₆-alkyl-, C₁-C₆-haloalkoxy-C₁-C₆-alkyl-, C₂-C₆-alkenyl-, C₂-C₆-alkynyl-, C₃-C₆-cycloalkyl-C₁-C₆-alkyl-, C₁-C₆-alkylcarbonyl-, C₁-C₆-alkoxycarbonyl- or aminocarbonyl-substituted aryl, heteroaryl, heterocyclyl, heterocyclyl-C₁-C₆-alkyl, aryl-C₁-C₆-alkyl, heteroaryl-C₁-C₆-alkyl, or NR⁵R⁶ in which R⁵ and R⁶ are each independently a radical from the group of hydrogen, C₁-C₆-alkyl, C₁-C₆-haloalkyl, C₃-C₆-cycloalkyl, C₁-C₆-alkoxy, C₁-C₆-alkylcarbonyl, C₁-C₆-alkoxylcarbonyl, hetaryl and heterocyclyl, or R⁵ and R⁶ together with the nitrogen atom to which they are bonded form an optionally halogen-substituted heterocycle,
R³ and R⁴ may also, together with the nitrogen atom to which they are bonded, form an optionally C₁-C₆-alkyl-, C₁-C₆-alkoxy- or halogen-substituted 3- to 7-membered ring optionally containing one or two heteroatoms from the group of oxygen, nitrogen and sulphur, where oxygen atoms must not be immediately adjacent, and salts, N-oxides and tautomeric forms of the compounds of the formula (I), where
halogen is selected from the group of fluorine, chlorine, bromine and iodine,
aryl (including as part of a larger unit, for example arylalkyl) is selected from the group of phenyl, naphthyl, anthryl, phenanthrenyl, and
hetaryl (synonymous with heteroaryl, including as part of a larger unit, for example hetarylalkyl) is selected from the group of furyl, thienyl, pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl,
for controlling animal pests.

2. Compounds of the formula (I) 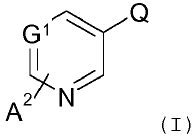 in which
G¹ is C-A¹,
A¹ is hydrogen,
A² is hydrogen,
Q is one of the following radicals 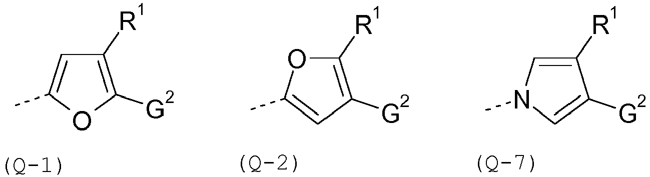
R¹ is hydrogen, C₁-C₄-alkyl, C₃-C₆-cycloalkyl, halogen, cyano, C₁-C₃-haloalkyl, hydroxyl or C₁-C₄-alkoxy,
G² is C (=X) NR³R⁴,
X and Y are each independently oxygen or sulphur,
R³ is a radical from the group of hydrogen, C₁-C₄-alkyl, C₃-C₆-cycloalkyl, C₁-C₄-haloalkyl, cyano-C₁-C₄-alkyl, C₁-C₄-alkoxy, C₁-C₄-haloalkoxy, C₂-C₄-alkenyl, C₁-C₄-alkoxy-C₁-C₄-alkyl, optionally halogen-substituted C₁-C₄-alkylcarbonyl, optionally halogen-substituted C₁-C₄-alkoxycarbonyl, optionally halogen-, C₁-C₄-alkyl-, C₁-C₄-alkoxy-, C₁-C₄-haloalkyl- and cyano-substituted C₃-C₆-cycloalkylcarbonyl, or a cation, for example a mono- or divalent metal ion or an optionally C₁-C₄-alkyl- or aryl-C₁-C₄-alkyl-substituted ammonium ion,
R⁴ is a radical from the group of hydrogen, cyano, in each case optionally halogen-, cyano-, C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-haloalkylsulphinyl-, C₁-C₄-alkylsulphonyl- and C₁-C₄-haloalkylsulphonyl-substituted C₁-C₄-alkyl, C₂-C₄-alkenyl and C₂-C₄-alkynyl, C₁-C₄-alkoxy, C₁-C₄-haloalkoxy, optionally halogen-substituted bis(C₁-C₄-alkoxy)-C₁-C₄-alkyl, optionally halogen-substituted bis (C₁-C₄-alkylthio)-C₁-C₄-alkyl, optionally halogen-substituted C₁-C₄-alkylcarbonyl-C₁-C₄-alkyl, optionally halogen-substituted C₁-C₄-alkylsulphinyl-C₁-C₄-alkyl, optionally halogen-substituted C₁-C₄-alkylsulphonyl-C₁-C₄-alkyl, optionally halogen-substituted C₁-C₄-alkoxycarbonyl-C₁-C₄-alkyl, C₂-C₄-alkynyloxy, optionally halogen-substituted C₃-C₆-cycloalkylcarbonyl, optionally halogen-substituted C₃-C₆-cycloalkyl-C₁-C₄-alkyl, in each case optionally halogen-, C₁-C₄-alkyl-, C₁-C₄-haloalkyl-, C₁-C₄-alkoxy- or C₁-C₄-haloalkoxy-substituted C₃-C₆-cycloalkyl and C₃-C₆-cycloalkenyl in which the rings may contain one to three heteroatoms from the group of sulphur, oxygen (where oxygen atoms must not be immediately adjacent) and nitrogen, in each case optionally halogen-, cyano- (including in the alkyl moiety), nitro-, C₁-C₄-alkyl-, C₁-C₄-haloalkyl-, C₃-C₆-cycloalkyl-, C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-haloalkylsulphinyl-, C₁-C₄-alkylsulphonyl-, C₁-C₄-haloalkylsulphonyl-, amino-, C₁-C₄-alkylamino-, di (C₁-C₄-alkyl) amino-, C₁-C₄-alkylcarbonylamino-, C₁-C₄-alkoxycarbonylamino-, C₁-C₄-alkoxy-C₁-C₄-alkyl-, C₁-C₄-haloalkoxy-C₁-C₄-alkyl-, C₂-C₄-alkenyl-, C₂-C₄-alkynyl-, C₃-C₆-cycloalkyl-C₁-C₄-alkyl-, C₁-C₄-alkylcarbonyl-, C₁-C₄-alkoxycarbonyl- or aminocarbonyl-substituted aryl, heteroaryl, aryl-C₁-C₄-alkyl, heteroaryl-C₁-C₄-alkyl, or NR⁵R⁶ in which R⁵ and R⁶ are each independently a radical from the group of hydrogen, C₁-C₄-alkyl, C₁-C₄-haloalkyl, C₃-C₆-cycloalkyl, C₁-C₄-alkoxy, C₁-C₄-alkylcarbonyl, C₁-C₄-alkoxylcarbonyl, hetaryl and heterocyclyl, or R⁵ and R⁶ together with the nitrogen atom to which they are bonded form an optionally halogen-substituted heterocycle,
R³ and R⁴ may also, together with the nitrogen atom to which they are bonded, form an optionally C₁-C₄-alkyl-, C₁-C₄-alkoxy- or halogen-substituted 3- to 7-membered ring optionally containing one or two heteroatoms from the group of oxygen, nitrogen and sulphur, where oxygen atoms must not be immediately adjacent,
and where
halogen is selected from the group of fluorine, chlorine,
bromine and iodine,
aryl (including as part of a larger unit, for example arylalkyl) is selected from the group of phenyl, naphthyl, anthryl, phenanthrenyl, and
hetaryl (including as part of a larger unit, for example hetarylalkyl) is selected from the group of thienyl, pyrimidyl, oxadiazolyl, oxazolyl, pyrazinyl, imidazolyl, thiazolyl and furanyl.

3. Compounds of the formula (I) according to Claim 2, in which
G¹ is C-A¹,
A¹ is hydrogen,
A² is hydrogen,
Q is Q-1, Q-2 or Q-7,
R¹ is hydrogen or C₁-C₄-alkyl,
G² is C (=X) NR³R⁴,
X is oxygen,
R³ is a radical from the group of hydrogen and C₁-C₄-alkyl,
R⁴ is a radical from the group of hydrogen, in each case optionally halogen-, cyano- and C₁-C₄-alkylthio-substituted C₁-C₄-alkyl and C₂-C₄-alkenyl, is C₂-C₄-alkynyl, optionally halogen-substituted C₃-C₆-cycloalkyl-C₁-C₄-alkyl and optionally C₁-C₄-haloalkyl-substituted heteroaryl-C₁-C₄-alkyl, and
R³ and R⁴ may also, together with the nitrogen atom to which they are bonded, form a 3- to 7-membered ring which does not contain any further heteroatoms, and where
halogen is selected from the group of fluorine, chlorine, bromine and iodine, and
hetaryl (including as part of a larger unit, for example hetarylalkyl) is selected from the group of thienyl, pyrimidyl and thiazolyl.

4. Method for controlling animal pests, **characterized in that** a compound of the formula (I) according to Claim 2 or 3 is allowed to act on the pests and/or their habitat, excluding methods implemented on the animal or human body.

## Revendications

1. Utilisation de composés de formule (I) 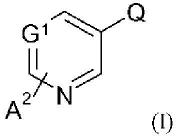 dans laquelle
G¹ représente C-A¹,
A¹ représente hydrogène,
A² représente hydrogène,
Q représente un des radicaux suivants 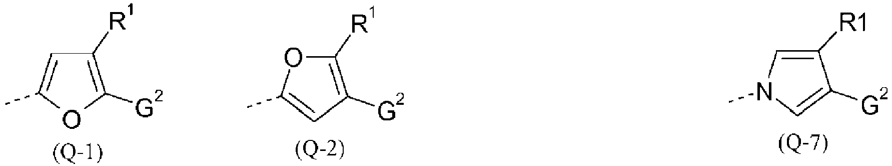
R¹ représente hydrogène, C₁-C₆-alkyle, C₃-C₆-cycloalkyle, halogéno, cyano, C₁-C₃-halogénoalkyle, hydroxy ou C₁-C₆-alcoxy,
G² représente C(=X)NR³R⁴,
X représente oxygène ou soufre,
R³ représente un radical de la série hydrogène ; C₁-C₆-alkyle ; C₃-C₆-cycloalkyle ; C₁-C₆-halogénoalkyle ; cyano-C₁-C₆-alkyle ; C₁-C₆-alcoxy ; C₁-C₆-halogénoalcoxy ; C₂-C₆-alcényle; C₁-C₆-alcoxy-C₁-C₆-alkyle C₁-C₆-alkylcarbonyle le cas échéant substitué par halogène ; C₁-C₆-alcoxycarbonyle le cas échéant substitué par halogène ; C₃-C₆-cycloalkylcarbonyle le cas échéant substitué par halogène, C₁-C₆-alkyle, C₁-C₆-alcoxy, C₁-C₆-halogénoalkyle et cyano ; ou un cation, tel que par exemple un ion métallique monovalent ou divalent ou un ion d'ammonium le cas échéant substitué par C₁-C₆-alkyle ou aryl-C₁-C₆-alkyle,
R⁴ représente un radical de la série hydrogène ; cyano ; C₁-C₆-alkyle, C₂-C₆-alcényle et C₂-C₆-alcynyle, C₁-C₆-alcoxy; C₁-C₆-halogénoalcoxy à chaque fois le cas échéant substitué par halogène, cyano, C₁-C₆-alcoxy, C₁-C₆-halogénoalcoxy, C₁-C₆-alkylthio, C₁-C₆-halogénoalkylthio, C₁-C₆-alkylsulfinyle, C₁-C₆-halogénoalkylsulfinyle, C₁-C₆-alkylsulfonyle et C₁-C₆-halogénoalkylsulfonyle ; bis (C₁-C₆-alcoxy) -C₁-C₆-alkyle le cas échéant substitué par halogène ; bis(C₁-C₆-alkylthio)-C₁-C₆-alkyle le cas échéant substitué par halogène ; C₁-C₆-alkylcarbonyl-C₁-C₆-alkyle le cas échéant substitué par halogène ; C₁-C₆-alkylsulfinyl-C₁-C₆-alkyle le cas échéant substitué par halogène ; C₁-C₆-alkylsulfonyl-C₁-C₆-alkyle le cas échéant substitué par halogène ; C₁-C₆-alcoxycarbonyl-C₁-C₆-alkyle le cas échéant substitué par halogène ; C₂-C₄-alcynyloxy ; C₃-C₆-cycloalkylcarbonyle le cas échéant substitué par halogène ; C₃-C₆-cycloalkyl-C₁-C₆-alkyle le cas échéant substitué par halogène ; C₃-C₆-cycloalkyle et C₃-C₆-cycloalcényle, dans lesquels les cycles peuvent contenir un à trois hétéroatomes de la série soufre, oxygène (des atomes d'oxygène ne pouvant pas être directement adjacents) et azote, à chaque fois le cas échéant substitué par halogène, C₁-C₆-alkyle, C₁-C₆-halogénoalkyle, C₁-C₆-alcoxy ou C₁-C₆-halogénoalcoxy ; aryle, hétéroaryle, hétérocyclyle, hétérocyclyl-C₁-C₆-alkyle, aryl-C₁-C₆-alkyle, hétéroaryl-C₁-C₆-alkyle à chaque fois le cas échéant substitué par halogène, cyano (également dans la partie alkyle), nitro, C₁-C₆-alkyle, C₁-C₆-halogénoalkyle, C₃-C₆-cycloalkyle, C₁-C₆-alcoxy, C₁-C₆-halogénoalcoxy, C₁-C₆-alkylthio, C₁-C₆-halogénoalkyl thio, C₁-C₆-alkylsulfinyle, C₁-C₆-halogénoalkylsulfinyle, C₁-C₆-alkylsulfonyle, C₁-C₆-halogénoalkylsulfonyle, amino, C₁-C₆-alkylamino, di(C₁-C₆-alkyl)amino, C₁-C₆-alkylcarbonylamino, C₁-C₆-alcoxycarbonylamino, C₁-C₆-alcoxy-C₁-C₆-alkyle, C₁-C₆-halogénoalcoxy-C₁-C₆-alkyle, C₂-C₆-alcényle, C₂-C₆-alcynyle, C₃-C₆-cycloalkyl-C₁-C₆-alkyle, C₁-C₆-alkylcarbonyle, C₁-C₆-alcoxycarbonyle ou aminocarbonyle ; ou NR⁵R⁶, où R⁵ et R⁶ représentent, indépendamment l'un de l'autre, un radical de la série hydrogène, C₁-C₆-alkyle, C₁-C₆-halogénoalkyle, C₃-C₆-cycloalkyle, C₁-C₆-alcoxy, C₁-C₆-alkylcarbonyle, C₁-C₆-alcoxylcarbonyle, hétaryle et hétérocyclyle ou R⁵ et R⁶ forment, ensemble avec l'atome d'azote auquel ils sont liés, un hétérocycle le cas échéant substitué par halogène,
R³ et R⁴ peuvent également former, ensemble avec l'atome d'azote auquel ils sont liés, un cycle de 3 à 7 chaînons, le cas échéant substitué par C₁-C₆-alkyle, C₁-C₆-alcoxy ou halogène et contenant le cas échéant un ou deux hétéroatomes de la série oxygène, azote et soufre, des atomes d'oxygène ne pouvant pas être directement adjacents,
ainsi que de sels, de N-oxydes et de formes tautomères des composés de formule (I),
halogène étant choisi dans la série fluor, chlore,
brome et iode,
aryle (également comme partie d'une unité plus grande, telle que par exemple arylalkyle) étant choisi dans la série phényle, naphtyle, anthryle, phénanthrényle et hétaryle (équivalent à hétéroaryle, également comme partie d'une unité plus grande, telle que par exemple hétarylalkyle) étant choisi dans la série furyle, thiényle, pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzo-isofuryle, benzothiényle, benzo-isothiényle, indolyle, iso-indolyle, indazolyle, benzothiazolyle, benzo-isothiazolyle, benzoxazolyle, benzo-isoxazolyle, benzimidazolyle, 2,1,3-benzoxadiazolyle, quinoléinyle, isoquinoléinyle, cinnolinyle, phtalazinyle, quinazolinyle, quinoxalinyle, naphtyridinyle, benzotriazinyle, purinyle, ptéridinyle et indolizinyle, pour lutter contre des organismes animaux nuisibles.

2. Composés de formule (I) 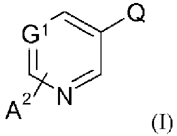 dans laquelle
G¹ représente C-A¹,
A¹ représente hydrogène,
A² représente hydrogène,
Q représente un des radicaux suivants 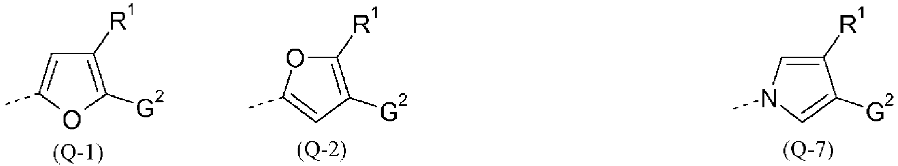
R¹ représente hydrogène ; C₁-C₄-alkyle ; C₃-C₆-cycloalkyle ; halogéno ; cyano ; C₁-C₃-halogénoalkyle ; hydroxy ; ou C₁-C₄-alcoxy,
G² représente C(=X)NR³R⁴,
X et Y représentent, indépendamment l'un de l'autre, oxygène ou soufre,
R³ représente un radical de la série hydrogène, C₁-C₄-alkyle, C₃-C₆-cycloalkyle, C₁-C₄-halogénoalkyle, cyano-C₁-C₄-alkyle, C₁-C₄-alcoxy, C₁-C₄-halogénoalcoxy, C₂-C₄-alcényle, C₁-C₄-alcoxy-C₁-C₄-alkyle ; C₁-C₄-alkylcarbonyle le cas échéant substitué par halogène ; C₁-C₄-alcoxycarbonyle le cas échéant substitué par halogène ; C₃-C₆-cycloalkylcarbonyle le cas échéant substitué par halogène, C₁-C₄-alkyle, C₁-C₄-alcoxy, C₁-C₄-halogénoalkyle et cyano ; ou un cation, tel que par exemple un ion métallique monovalent ou divalent ou un ion d'ammonium le cas échéant substitué par C₁-C₄-alkyle ou aryl-C₁-C₄-alkyle,
R⁴ représente un radical de la série hydrogène ; cyano ; C₁-C₄-alkyle, C₂-C₄-alcényle et C₂-C₄-alcynyle, C₁-C₄-alcoxy ; C₁-C₄-halogénoalcoxy à chaque fois le cas échéant substitué par halogène, cyano, C₁-C₄-alcoxy, C₁-C₄-halogénoalcoxy, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle et C₁-C₄-halogénoalkylsulfonyle ; bis (C₁-C₄-alcoxy) -C₁-C₄-alkyle le cas échéant substitué par halogène ; bis(C₁-C₄-alkylthio)-C₁-C₄-alkyle le cas échéant substitué par halogène ; C₁-C₄-alkylcarbonyl-C₁-C₄-alkyle le cas échéant substitué par halogène ; C₁-C₄-alkylsulfinyl-C₁-C₄-alkyle le cas échéant substitué par halogène ; C₁-C₄-alkylsulfonyl-C₁-C₄-alkyle le cas échéant substitué par halogène ; C₁-C₄-alcoxycarbonyl-C₁-C₄-alkyle le cas échéant substitué par halogène ; C₂-C₄-alcynyloxy ; C₃-C₆-cycloalkylcarbonyle le cas échéant substitué par halogène ; C₃-C₆-cycloalkyl-C₁-C₄-alkyle le cas échéant substitué par halogène ; C₃-C₆-cycloalkyle et C₃-C₆-cycloalcényle, dans lesquels les cycles peuvent contenir un à trois hétéroatomes de la série soufre, oxygène (des atomes d'oxygène ne pouvant pas être directement adjacents) et azote, à chaque fois le cas échéant substitué par halogène, C₁-C₄-alkyle, C₁-C₄-halogénoalkyle, C₁-C₄-alcoxy ou C₁-C₄-halogénoalcoxy ; aryle, hétéroaryle, aryl-C₁-C₄-alkyle, hétéroaryl-C₁-C₄-alkyle à chaque fois le cas échéant substitué par halogène, cyano (également dans la partie alkyle), nitro, C₁-C₄-alkyle, C₁-C₄-halogénoalkyle, C₃-C₆-cycloalkyle, C₁-C₄-alcoxy, C₁-C₄-halogénoalcoxy, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle, C₁-C₄-halogénoalkylsulfonyle, amino, C₁-C₄-alkylamino, di(C₁-C₄-alkyl)amino, C₁-C₄-alkylcarbonylamino, C₁-C₄-alcoxycarbonylamino, C₁-C₄-alcoxy-C₁-C₄-alkyle, C₁-C₄-halogénoalcoxy-C₁-C₄-alkyle, C₂-C₄-alcényle, C₂-C₄-alcynyle, C₃-C₆-cycloalkyl-C₁-C₄-alkyle, C₁-C₄-alkylcarbonyle, C₁-C₄-alcoxycarbonyle ou aminocarbonyle ; ou NR⁵R⁶, où R⁵ et R⁶ représentent, indépendamment l'un de l'autre, un radical de la série hydrogène, C₁-C₄-alkyle, C₁-C₄-halogénoalkyle, C₃-C₆-cycloalkyle, C₁-C₄-alcoxy, C₁-C₄-alkylcarbonyle, C₁-C₄-alcoxylcarbonyle, hétaryle et hétérocyclyle ou R⁵ et R⁶ peuvent former, ensemble avec l'atome d'azote auquel ils sont liés, un hétérocycle le cas échéant substitué par halogène,
R³ et R⁴ peuvent également former, ensemble avec l'atome d'azote auquel ils sont liés, un cycle de 3 à 7 chaînons, le cas échéant substitué par C₁-C₄-alkyle, C₁-C₄-alcoxy ou halogène et contenant le cas échéant un ou deux hétéroatomes de la série oxygène, azote et soufre, des atomes d'oxygène ne pouvant pas être directement adjacents,
et
halogène étant choisi dans la série fluor, chlore,
brome et iode,
aryle (également comme partie d'une unité plus grande,
telle que par exemple arylalkyle) étant choisi dans la série phényle, naphtyle, anthryle, phénanthrényle et hétaryle (également comme partie d'une unité plus grande, telle que par exemple hétarylalkyle) étant choisi dans la série thiényle, pyrimidyle, oxadiazolyle, oxazolyle, pyrazinyle, imidazolyle, thiazolyle et furannyle.

3. Composés de formule (I) selon la revendication 2, où
G¹ représente C-A¹,
A¹ représente hydrogène,
A² représente hydrogène,
Q représente Q-1, Q-2 ou Q-7,
R¹ représente hydrogène ou C₁-C₄-alkyle,
G² représente C(=X)NR³R⁴,
X représente oxygène,
R³ représente un radical de la série hydrogène et C₁-C₄-alkyle,
R⁴ représente un radical de la série hydrogène ; C₁-C₄-alkyle et C₂-C₄-alcényle à chaque fois le cas échéant substitué par halogène, cyano et C₁-C₄-alkylthio ; C₂-C₄-alcynyle ; C₃-C₆-cycloalkyl-C₁-C₄-alkyle le cas échéant substitué par halogène ; et hétéroaryl-C₁-C₄-alkyle le cas échéant substitué par C₁-C₄-halogénoalkyle et
R³ et R⁴ peuvent également former, ensemble avec l'atome d'azote auquel ils sont liés, un cycle de 3 à 7 chaînons, qui ne contient pas d'autres hétéroatomes et
halogène étant choisi dans la série fluor, chlore, brome et iode et
hétaryle (également comme partie d'une unité plus grande, telle que par exemple hétarylalkyle) étant choisi dans la série thiényle, pyrimidyle et thiazolyle.

4. Procédé pour lutter contre les organismes animaux nuisibles, **caractérisé en ce qu'**on laisse agir des composés de formule (I) selon la revendication 2 ou 3 sur les organismes nuisibles et/ou leur espace de vie, les procédés sur le corps animal et humain étant exclus.
